# EUROPEAN PATENT APPLICATION

(11) **EP 4 555 915 A1**
(43) Date of publication of application: **21.05.2025**
(21) Application number: 23854489.4
(22) Date of filing: 17.08.2023
(51) Int. Cl.: A61B 1/06, A61B 1/04, G01J 1/42, G01J 1/04

(54) **LIGHT SOURCE APPARATUS AND ENDOSCOPIC SYSTEM**

(30) Priority: 17.08.2022 CN 202210989189; 17.08.2022 CN 202210988103; 17.08.2022 CN 202210989280
(71) Applicant: Changzhou United Imaging Surgical Co., Ltd., Changzhou, Jiangsu 213000 (CN)
(72) Inventor: DOH, Takeshi, hangzhou, Jiangsu 213000 (CN); LIU, Juanjuan, hangzhou, Jiangsu 213000 (CN)
(74) Representative: Wang, Bo
(86) International application number: PCT/CN2023/113538
(87) International publication number: WO 2024/037590

(57) **Abstract**

A light source device comprises a first light combination module and at least two light sources. The light source device is configured to be connected with a light guide module. The at least two light sources include a first light source and at least one second light source. The first light combination module includes a first light combination element. The first light combination element is disposed between the light guide module and the first light source and configured to transform a first beam into a first transmitted light, the first beam being related to the first light source. The first light combination element is further configured to generate a first reflected light from at least one second beam emitted by the at least one second light source, combine the first reflected light and the first transmitted light into a combined light, and send the combined light to a tissue through the light guide module. A spectrum of the first beam is narrowband or short-wavelength range.

## Description

### CROSS-REFERENCE TO RELATED APPLICATIONS

This application claims priority to Chinese Application No. 202210989189.6, filed on August 17, 2022, Chinese Application No. 202210988103.8, filed on August 17, 2022, and Chinese Application No. 202210989280.8, filed on August 17, 2022, the entire contents of each of which are incorporated herein by reference.

### TECHNICAL FIELD

The present disclosure relates to the field of endoscope technology, and in particular to a light source device and an endoscope system.

### BACKGROUND

**In** the field of medical imaging technology, it is common to use an endoscope system for diagnosis. A light source device used in the existing endoscope system generally uses a plurality of light-emitting diodes (LEDs) to combine light to output beams corresponding to an ordinary light observation mode or a special light (e.g., a special light in a narrow band or short band) observation mode. However, in the special light observation mode, a light flux of special light is limited due to a narrow bandwidth of the narrow band. Furthermore, a transmittance of a short-wave band light transmitted by a light guide module of the endoscope system is lower than a transmittance of a long-wave band light, which also leads to insufficient light flux of the short-wave beam in lighting of the endoscope system. The light flux of white light and special light affects a diagnostic result of the endoscope system. For example, when observed at a medium and long distance, the insufficient light flux of the special light limits the recognition of a living tissue, making it impossible to determine whether the living tissue has a lesion. Therefore, it is desirable to provide a light source device of an endoscope system and the endoscope system with sufficient light flux of special light and are easy to mount and adjust.

### SUMMARY

One or more embodiments of the present disclosure provide a light source device. The light source device may comprise a first light combination module and at least two light sources. The light source device may be configured to be connected with a light guide module. The at least two light sources may include a first light source and at least one second light source. The first light combination module may include a first light combination element. The first light combination element may be disposed between the light guide module and the first light source and configured to transform a first beam into a first transmitted light. The first beam may be related to the first light source. The first light combination element may be further configured to generate a first reflected light from at least one second beam emitted by the at least one second light source, combine the first reflected light and the first transmitted light into a combined light, and send the combined light to a tissue through the light guide module. The spectrum of the first beam is narrowband or short-wavelength range.

One or more embodiments of the present disclosure provide an endoscope system. The endoscope system may comprise light guide module, a lighting module, a camera module, a processing module, a display module, and the light source device. The light source device may be configured to input the combined light into the light guide module. The light guide module may be configured to transmit an input light to the lighting module. The lighting module may be configured to diffuse the combined light transmitted to the lighting module to a tissue. The camera module may be configured to obtain an image of the tissue. The processing module may be configured to perform signal processing on the image to obtain an image. The display module may be configured to display the image after the signal processing.

### BRIEF DESCRIPTION OF THE DRAWINGS

The present disclosure will be further illustrated by way of exemplary embodiments, which will be described in detail by means of the accompanying drawings. These embodiments are not limiting, and in these embodiments, the same numbering indicates the same structure, where:
FIG. 1 is a schematic diagram illustrating an exemplary endoscope system according to some embodiments of the present disclosure;
FIG. 2 is a schematic diagram illustrating an exemplary light source device according to some embodiments of the present disclosure;
FIG. 3 is a schematic diagram illustrating an exemplary light source device according to some embodiments of the present disclosure;
FIG. 4 is a schematic diagram illustrating an exemplary light source device according to some embodiments of the present disclosure;
FIG. 5 is a schematic diagram illustrating exemplary spectrum curves of light sources according to some embodiments of present disclosure;
FIG. 6 is a schematic diagram illustrating a transmittance spectrum of an exemplary first light combination element according to some embodiments of the present disclosure;
FIG. 7 is a schematic diagram illustrating a transmittance spectrum of an exemplary second light combination element according to some embodiments of the present disclosure;
FIG. 8 is a schematic diagram illustrating a transmittance spectrum of an exemplary second light combination element according to some embodiments of the present disclosure;
FIG. 9 is a schematic structural diagram illustrating an exemplary light source device according to some embodiments of the present disclosure;
FIG. 10 is a schematic diagram illustrating a transmittance spectrum of an exemplary second light combination element according to some embodiments of the present disclosure;
FIG. 11 is a schematic diagram illustrating a transmittance spectrum of an exemplary second light combination element according to some embodiments of the present disclosure;
FIG. 12 is a schematic structural diagram illustrating an exemplary light source device according to some embodiments of the present disclosure;
FIG. 13 is a schematic structural diagram illustrating an exemplary light source device according to some embodiments of the present disclosure;
FIG. 14 is a schematic structural diagram illustrating an exemplary light source device according to some embodiments of the present disclosure;
FIG. 15A is a schematic structural diagram illustrating an exemplary light source device according to some embodiments of the present disclosure;
FIG. 15B is a schematic structural diagram illustrating an exemplary light source device according to some embodiments of the present disclosure;
FIG. 15C is a schematic structural diagram illustrating an exemplary light source device according to some embodiments of the present disclosure;
FIG. 15D is a schematic diagram illustrating a coating of a first optical surface of an exemplary third dichroic mirror according to some embodiments of the present disclosure;
FIG. 15E is a schematic diagram illustrating positions of an exemplary first photodetector and an exemplary second photodetector according to some embodiments of the present disclosure;
FIG. 15F is a schematic diagram illustrating a position of an exemplary background light detector relative to a first photodetector and a second photodetector according to some embodiments of the present disclosure;
FIG. 15G is a schematic diagram illustrating a transmittance spectrum of an exemplary third dichroic mirror according to some embodiments of the present disclosure;
FIG. 16 is a schematic structural diagram illustrating an exemplary light source device according to some embodiments of the present disclosure;
FIG. 17 is a schematic structural diagram illustrating an exemplary light source device according to some embodiments of the present disclosure;
FIG. 18 is a schematic structural diagram illustrating an exemplary light source device according to some embodiments of present disclosure;
FIG. 19A is a schematic structural diagram illustrating an exemplary light source device according to some embodiments of present disclosure;
FIG. 19B is a schematic structural diagram illustrating an exemplary light source device according to some embodiments of present disclosure;
FIG. 19C is a schematic structural diagram illustrating an exemplary light source device according to some embodiments of present disclosure;
FIG. 19D is a schematic structural diagram illustrating an exemplary light source device according to some embodiments of present disclosure;
FIG. 19E is a schematic structural diagram illustrating an exemplary light source device according to some embodiments of present disclosure;
FIG. 19F is a schematic diagram illustrating a spectrum curve of a first optical surface of a dichroic mirror according to some embodiments of the present disclosure;
FIG. 20 is a schematic diagram illustrating exemplary spectrum curves of light sources according to some embodiments of the present disclosure;
FIG. 21 is a schematic diagram illustrating a transmittance spectrum of an exemplary third light combination element according to some embodiments of the present disclosure;
FIG. 22 is a schematic structural diagram illustrating an exemplary light source device according to some embodiments of the present disclosure;
FIG. 23 is a schematic diagram illustrating exemplary spectrum curves of light sources according to some embodiments of the present disclosure;
FIG. 24 is a schematic diagram illustrating a transmittance spectrum of an exemplary fourth light combination element according to some embodiments of the present disclosure;
FIG. 25 is a schematic diagram illustrating a transmittance spectrum of an exemplary fifth light combination element according to some embodiments of the present disclosure;
FIG. 26 is a schematic structural diagram illustrating an exemplary light source device according to some embodiments of the present disclosure;
FIG. 27 is a schematic structural diagram illustrating an exemplary light source device according to some embodiments of the present disclosure;
FIG. 28 is a schematic diagram illustrating exemplary spectrum curves of light sources according to some embodiments of the present disclosure;
FIG. 29 is a schematic diagram illustrating a transmittance spectrum of an exemplary light combination element 264 according to some embodiments of the present disclosure;
FIG. 30 is a schematic structural diagram illustrating an exemplary light source device according to some embodiments of the present disclosure.

### DETAILED DESCRIPTION

In order to more clearly illustrate the technical solutions of the embodiments of the present disclosure, the accompanying drawings required to be used in the description of the embodiments are briefly described below. Obviously, the accompanying drawings in the following description are only some examples or embodiments of the present disclosure, and it is possible for a person of ordinary skill in the art to apply the present disclosure to other similar scenarios in accordance with these drawings without creative labor. Unless obviously obtained from the context or the context illustrates otherwise, the same numeral in the drawings refers to the same structure or operation.

It should be understood that the terms "system," "device," "unit," and/or "module" used herein are a way to distinguish between different components, elements, parts, sections, or assemblies at different levels. However, the terms may be replaced by other expressions if other words accomplish the same purpose.

In the present disclosure, the terms "first," and "second" are used for descriptive purposes only and should not be understood as indicating or implying relative importance or implicitly indicating the number of technical features indicated. Thus, a feature defined as "first," or "second" may explicitly or implicitly include at least one of the features. In the description of this application, the meaning of "plurality" is at least two, for example, two, three, etc., unless otherwise clearly and specifically defined.

In the present disclosure, unless otherwise clearly specified and limited, the terms "mounting," "connecting," "connection," "fixing", or the like, should be understood in a broad sense, for example, it can be a fixed connection, a detachable connection, or an integral connection; it can be a mechanical connection or an electrical connection; it can be a direct connection or an indirect connection through an intermediate medium, it can be an internal connection of two elements or an interaction relationship between two elements, unless otherwise clearly defined. For those having ordinary skills in the art, the specific meanings of the above terms in this application can be understood according to specific circumstances.

As shown in the present disclosure and in the claims, unless the context clearly suggests an exception, the words "one," "a," "an," "one kind," and/or "the" do not refer specifically to the singular, but may also include the plural. Generally, the terms "including," and "comprising" suggest only the inclusion of clearly identified steps and elements, however, the steps and elements that do not constitute an exclusive list, and the method or apparatus may also include other steps or elements.

Flowcharts are used in the present disclosure to illustrate the operations performed by a system according to embodiments of the present disclosure, and the related descriptions are provided to aid in a better understanding of the magnetic resonance imaging method and/or system. It should be appreciated that the preceding or following operations are not necessarily performed in an exact sequence. Instead, steps can be processed in reverse order or simultaneously. Also, it is possible to add other operations to these processes or to remove a step or steps from these processes.

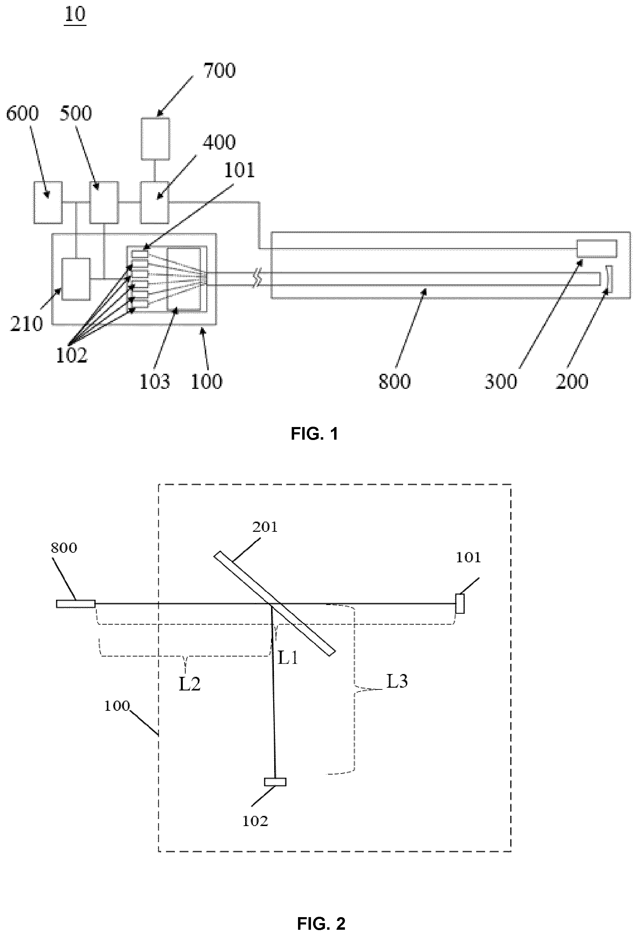

FIG. 1 is a schematic diagram illustrating an exemplary endoscope system 10 according to some embodiments of the present disclosure. As shown in FIG. 1, the endoscope system 10 may include a light source device 100, a lighting module 200, a camera module 300, a processing module 400, a control module 500, an input module 600, a display module 700, and a light guide module 800. It should be understood that the endoscope system 10 shown in FIG. 1 can be applied to the medical field. By inserting the endoscope system 10 into a body cavity, such as the esophagus, the stomach, and the large intestine for observation and diagnosis, and providing lighting for internal observation through the light source device 100, the endoscope system 10 is widely used in the discovery, diagnosis, and treatment of a lesion. For example, the light source device 100 may provide an ordinary white light to observe overall properties of a surface of a living tissue, and obtain high-contrast enhanced images of different subjects according to the absorption, reflection, and scattering characteristics of mucosa and blood vessels. As another example, the light source device 100 may provide a blue narrowband and/or green narrowband light to enhance the contrast between capillaries on the surface of the mucosa and thick blood vessels deep inside, which is helpful for lesion screening.

In some embodiments, the light source device 100 may be configured to provide lighting for the endoscope system 10. In some embodiments, the light source device 100 may include at least two light sources and a combination component for combining lights emitted by the at least two light sources. As shown in FIG. 1, the light source device 100 may include a first light source 101, at least one second light source 102, and a first light combination module 103. The first light combination module 103 may be configured to combine lights emitted by the first light source 101 and the at least one second light source 102 to generate a combined light. In some embodiments, the light source device 100 may further include a heat dissipation module 210 for heat dissipation from the light source device 100 to make the light source device 100 operate within a reasonable temperature range. For example, the heat dissipation module 210 may be configured to cool the light source device 100. In some embodiments, the heat dissipation module 210 may allow air to circulate or generate cold air, and use the air or the cold air to exchange heat with other structures for cooling.

In some embodiments, the light source device 100 may further include a photodetector for detecting a light flux of each light source under a preset driving current. In some embodiments, the processing module 400 may obtain a detection signal of the light flux of each light source, and adjust a driving current (and/or a driving voltage) of each light source according to a difference between the detection signal and a preset detection signal. When the preset detection signal is used to calibrate the detection signal of the photodetector, a corresponding relationship between different driving currents, detection signals, and light fluxes of a plurality of light sources (e.g., N light sources, numbered 11-1N) may be established. During calibration, a driving current I1-IN of each light source 11-1N may be changed or increased point by point, and a beam splitter in a collimating optical path of each light source 11-1N may split a beam to be incident into the photodetector. The photodetector may detect a light flux φ1-φN of each photodetector under the corresponding driving current, and convert a light flux signal φ1-φN into the detection signal L1-LN. In this way, the corresponding relationship between the driving current, the detection signal, and the light flux is Ii: Li: φi (i=1-N). After multi-point testing, a relationship curve of the driving current, the detection signal, and the light flux may be obtained, the calibration may be completed, and a calibration result may be stored (e.g., stored in the processing module 400). In some embodiments, feedback control of the light flux output by each light source may be accurately achieved according to the calibration result. For example, if an actual detection signal of a light source is less than the preset detection signal, the control module 500 may increase a driving current corresponding to the light source; if the actual detection signal of the light source is greater than the preset detection signal, the control module 500 may reduce the driving current corresponding to the light source. More descriptions regarding the light source device 100 and internal components thereof may be found in FIGs. 2-4, FIG. 9, FIGs. 12-19, and FIG. 22 of the present disclosure.

Since a hue stability of the combined light of the light source device 100 has a significant impact on the observation of a diseased tissue, the brightness of the combined light has an important impact on the intensity of an image signal (e.g., a clarity of an output image). Through the real-time signal detection of the photodetector and the calibration result, the feedback control of the light output by each light source can be accurately achieved, thereby maintaining the stability of the illumination light hue and the stability of the light flux, and providing the illumination light required by the camera module 300.

In some embodiments, as shown in FIG. 1, the light guide module 800 may be respectively connected with the light source device 100 and the lighting module 200. The light guide module 800 is configured to transmit the combined light provided by the light source device 100 to the lighting module 200. In some embodiments, the lighting module 200 may be configured to diffuse the combined light provided by the light source device 100 to a target subject (e.g., a tissue of the human body), thereby providing diagnosis or treatment for the target subject. For example, the lighting module 200 may include a lighting lens. The lighting lens may be configured to diffuse the light provided by the light source device 100 to the target subject. As another example, the lighting module 200 may include a concave lens, and the beam may be diffused by the concave lens. As another example, the light guide module 800 may include an optical fiber.

In some embodiments, the camera module 300 may be configured to acquire an image of the target subject and transmit the image of the target subject to the processing module 400. In some embodiments, the processing module 400 may be configured to perform signal processing on the image acquired by the camera module 300 to obtain an image after signal processing, and transmit the image after signal processing to the display module 700. In some embodiments, the display module 700 may be configured to display the image after signal processing by the processing module 400.

In some embodiments, the input module 600 may be configured to obtain an input instruction for controlling the light source device 100. For example, the input instruction may include an operation instruction for any one of an ordinary white light mode, a special light mode, and a hybrid light mode. It should be understood that the "ordinary white light mode" in the present disclosure refers to outputting a white light hue illumination light by proportionally controlling various light source components in the light source device 100 to obtain an image of a living tissue through the endoscope system 10. The "special light mode" refers to at least one special light source, such as a violet light source, a blue light source, or a green light source, etc. Different wavelengths have different incident depths in the living tissue. For example, the longer the wavelength, the deeper the incident depth in the living tissue. The high absorption of blood vessels at different depths in the surface and middle layers is contrasted with the low absorption of the mucosa, and the high-contrast images of blood vessels at different depths are obtained through the endoscope system 10. The "hybrid light mode" is different from the ordinary white light mode and the special light mode, and refers to a spectrum output different from the ordinary white light mode and the special light mode obtained based on a partial spectrum with the special light mode and a partial spectrum with the ordinary white light mode. An image that considers an overall contour of the living tissue and emphasizes observation of the blood vessels may be achieved through the endoscope system 10.

In some embodiments, the light source device 100 may be connected with a first end of the control module 500. A second end of the control module 500 may be connected with the input module 600. A third end of the control module 500 may be connected with a first end of the processing module 400. A second end of the processing module 400 may be connected with the camera module 300. In some embodiments, the control module 500 may be configured to control a light mode of the combined light emitted by the light source device 100 according to the light mode in the input instruction. For example, when the input instruction received by the input module 600 is any one of the ordinary white light mode, the special light mode, and the hybrid light mode, the control module 500 may control the light mode of the combined light emitted by the light source device 100 based on the light mode in the input instruction to complete switching between the ordinary white light mode, the hybrid light mode, or the special light mode.

In some embodiments, the control module 500 may adjust the driving current (or the driving voltage) of each light source of the light source device 100, adjust a change of the light flux output from each light source, or change the light flux by adjusting a current pulse width modulation (PWM). Or the control module 500 may control an operation state of the light source device 100 and the camera module 300. For example, a light flux ratio output from each light source may be controlled according to a preset light flux ratio to achieve a corresponding illumination light mode. The light flux output from each light source may be adjusted as a whole according to a brightness level of imaging of the camera module 300. The photodetector may be disposed in the light source device 100 to realize the real-time feedback control of each light source component in the output light, so as to maintain the hue stability of the illumination light and the stability of the light flux, provide the illumination light required by the camera module 300, and simplify the light flux control strategy.

In some embodiments, the input instruction may further include starting cooling and stopping cooling. After the input module 400 obtains the input instruction for starting cooling, the control module 500 may control the heat dissipation module 210 to turn on. After the input module 400 obtains the input instruction for stopping cooling, the control module 500 may control the heat dissipation module 210 to turn off. In some embodiments, the input instruction may further include automatic control information generated by the processing module 600. After the processing module 600 generates the automatic control information, the processing module 600 may sent the automatic control information to the control module 500, and the control module 500 may perform a corresponding control function according to the automatic control information. The automatic control information may include at least one or more of control information for automatically controlling an operation mode of the first light combination module 103, control information for automatically controlling the driving current, and control information for automatically controlling the heat dissipation module 210.

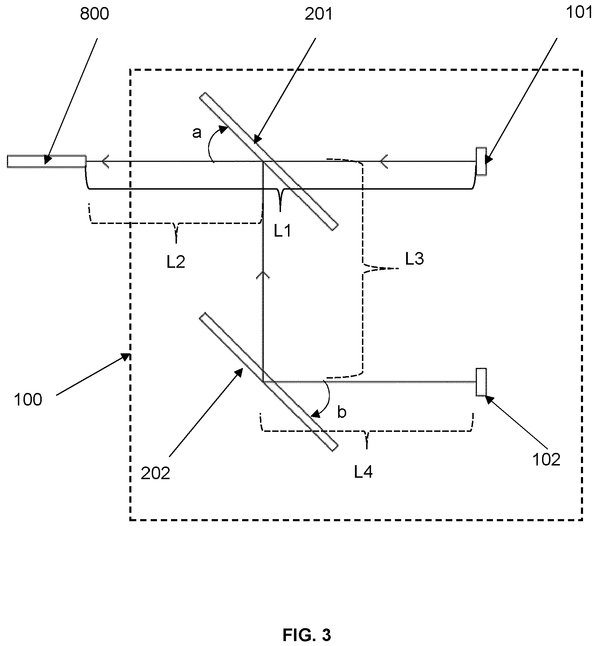

FIG. 2 is a schematic diagram illustrating an exemplary light source device 100 according to some embodiments of the present disclosure. FIG. 3 is a schematic diagram illustrating an exemplary light source device 100 according to some embodiments of the present disclosure. As shown in FIG. 1, the light source device 100 may include at least two light sources (e.g., the at least two light sources may include a first light source 101 and at least one second light source 102) and the first light combination module 103. As shown in FIGs. 2-3, the light guide module 800 may be connected with the light source device 100 and configured output the combined light generated by the light source device 100. The at least two light sources may include the first light source 101 and the at least one second light source 102.

In some embodiments, the first light source 101, the at least one second light source 102, and the first light combination module 103 may be detachably arranged in a same module to form a modular light source device 100, so as to facilitate replacement, assembly and disassembly of different first light sources 101, the at least one second light source 102, or the first light combination module 103 as needed. In some embodiments, the light guide module 800 may be detachably connected with the light source device 100.

The at least two light sources of the light source device 100 may be configured to provide lighting for the endoscope system. In some embodiments, the at least two light sources may include the first light source 101 and the at least one second light source 102. The first light source 101 and each of the at least two second light sources 102 may emit a light of a different frequency range, respectively. In some embodiments, the first light source 101 may include a narrowband light source or a short-wave light source. The "narrowband light source" refers to a light source of which a bandwidth is less than 50 nm. Correspondingly, "narrowband" refers to a band of which a bandwidth is less than 50 nm, and a band of which a bandwidth is greater than 50 nm is referred to as a "broadband". The "short-wave light source" refers to a portion of a commonly used illumination spectrum range 370 nm-780 nm that is close to a short-wave range. For example, the short-wave light source refers to a light source of which a wavelength is in a range of 370 nm-460 nm. As another example, the short-wave light source refers to a light source of which a wavelength is in a range of 400 nm-610 nm. As another example, the short-wave light source refers to a light source of which a wavelength is in a range of 380 nm-500 nm. In some embodiments, a peak wavelength of the first light source 101 may be in a range of 370 nm-650 nm. For example, the first light source 101 may include a violet light source of which a peak wavelength is in a range of 370 nm-430 nm, a blue light source of which a peak wavelength is in a range of 430 nm-460 nm (e.g., a narrowband blue light source), a green light source of which a peak wavelength is in a range of 510 nm-560 nm (e.g., a narrowband green light source), an amber light source of which a peak wavelength is in a range of 590 nm-610 nm (e.g., a narrowband amber light source), a red light source of which a peak wavelength is in a range of 620 nm-650 nm (e.g., a narrowband red light source), or any combination thereof. In some embodiments, the at least one second light source 102 may serve as a complementary light source of the first light source to be combined with the first light source to output the illumination light (such as a white light with a color temperature and a color rendering index matching a xenon lamp) required for the endoscope system. The at least one second light source 102 may be a broadband light source or a narrowband light source, or a combination thereof. It should be understood that the broadband light source refers to a light source with a bandwidth greater than 50 nm compared with the narrowband light source. Merely by way of example, the first light source 101 may be a violet light source, and the at least one second light source 102 may be a white light source.

In some embodiments, each light source may be a solid light source. For example, each light source may be a light emitting diode (LED) or a laser diode (LD), or a fluorescent light source using the LED or the LD as an excitation light source (such as a green fluorescent LED or LD). In some embodiments, a light emitted by the first light source 101 has a narrowband spectrum. For example, the first light source 101 may be a violet light source with a bandwidth less than or equal to 20 nm. As another example, a spectrum of the light emitted by the first light source 101 is not the narrowband, but may be filtered by a filter to obtain a narrowband spectrum. For example, the first light source 101 may be a green light source with a bandwidth of about 100 nm, which may reach a narrowband green light of less than or equal to 50 nm after narrowband filtering. In some embodiments, when the bandwidth of a beam emitted by the narrowband light source is greater than a bandwidth of a light source required by the endoscope system, narrowband processing may be performed on the beam emitted by the narrowband light source to obtain the bandwidth required by the endoscope system. For example, a filter may be disposed between the narrowband light source and the first light combination module 103 for narrowband filtering to obtain the light source required by the endoscope system. Furthermore, the filter may be controlled by a switch-in and switch-out structure to achieve two bandwidth outputs of the broadband light and the narrowband light.

The first light combination module 103 may be configured to perform optical path integration on at least two beams generated by the at least two light sources to output the combined light. In some embodiments, the first light combination module 103 may include one or more light combination elements. The one or more light combination elements may be disposed between the light guide module 800 and the at least two light sources. In some embodiments, the first light combination module 103 may include the one or more light combination elements for reflecting a beam of a partial band and transmitting a beam of another band. The one or more light combination elements may be arranged and combined at different positions to control a path of the beam. For example, the one or more light combination elements may include a dichroic mirror (e.g., a long-wave pass dichroic mirror, a short-wave pass dichroic mirror, or a band-pass dichroic mirror), a light combination prism, etc. In some embodiments, the first light combination module 103 may include at least one light combination element. As shown in FIG. 2, the first light combination module 103 may include a first light combination element 201. As shown in FIG. 3, the first light combination module 103 may include the first light combination element 201 and at least one second light combination element 202.

In some embodiments, the first light source 101 may include the narrowband light source or the short-wave light source. In some embodiments, in order to increase the light flux of the narrowband light source or the short-wave light source, a reflection optical path of the narrowband light source or the short-wave light source may be reduced. Meanwhile, in order to make the internal components of the light source device 100 easy to adjust, the light source (e.g., the narrowband light source or the short-wave light source) emitted by the first light source 101 may be directly transmitted. For example, as shown in FIG. 2 and FIG. 3, the first light combination element 201 may be disposed between the light guide module 800 and the first light source 101, and configured to transform a first beam into a first transmitted light. The spectrum of the first beam is narrowband or short-wavelength range. In some embodiments, the first beam may be related to the first light source 101. In the present disclosure, the first beam being related to the first light source 101 refers that the first beam is related to the light emitted by the first light source 101. For example, when the first light source 101 is the narrowband light source or the short-wave light source, the first beam may be the light directly emitted by the first light source 101. For example, the first beam may belong to a narrowband spectrum range or a short-waveband spectrum range. As another example, when the first light source 101 is a broadband spectrum, the first beam may be a narrowband spectrum or a short-wave spectrum formed by the light emitted by the first light source 101 after narrowband filtering. In some embodiments, the first beam may be directly transmitted through the first light combination element 201 without deflection of the reflection optical path, and transmitted to a tissue through the light guide module 800. Therefore, the optical transmission efficiency of the first light source 101 is not easily affected by the assembly accuracy, such that a relatively high optical efficiency can be achieved, and the problem of insufficient light flux of the first beam due to the narrowband characteristics or attenuation of the transmittance of the short-wave light transmitted by the light guide module can be solved, thereby improving the light flux of the first beam.

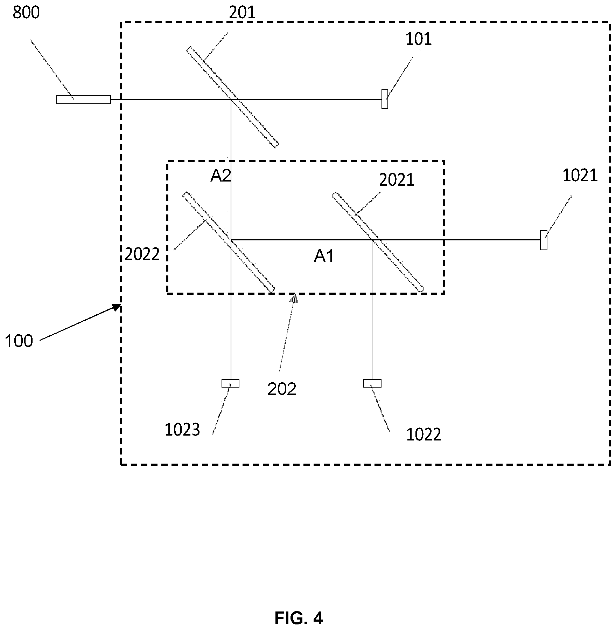

In some embodiments, as shown in FIG. 2 or FIG. 3, the first light combination element 201 may be further configured to generate a first reflected light from at least one second beam emitted by the at least one second light source 102, combine the first reflected light and the first transmitted light into a combined light, and send the combined light to the tissue by the light guide module 800. In some embodiments, when the at least one second light source 102 includes only one second light source 102, the first light combination element 201 may be configured to reflect the second beam emitted by the second light source 102 to form the first reflected light (as shown in FIG. 2). In some embodiments, when the at least one second light source 102 includes two or more second light sources 102, the at least one second light combination element 202 may be configured to combine a plurality of second beams emitted by the two or more second light sources 102, and then reflect the combined light by the first light combination element 201 to form the first reflected light (as shown in FIG. 4, FIG. 9, FIGs. 12-19, and FIG. 22).

In some embodiments, the first light combination module 103 may include the first light combination element 201 and the at least one second light combination element 202. When a count N of the at least one second light source 102 is at least two, the first light combination module 103 may include at least N-1 second light combination elements 202. Each of the N-1 second light combination elements may be configured to combine a second beam emitted by each of the at least one second light source 102 after the second beam being reflected and/or transmitted to generate a first incident beam, and the first incident beam may be incident onto the first light combination element for reflection. In some embodiments, as shown in FIG. 4, when the count of the at least one second light source 102 is three (including a second light source 1021, a second light source 1022, and a second light source 1023), the first light combination module 103 may include two second light combination elements 202 (as shown in FIG. 4, a second light combination element 2021 and a second light combination element 2022). The second light combination element 2021 may be configured to combine beams emitted by the second light source 1021 and the second light source 1022 by transmitting and/or reflecting the beams respectively, and the second light combination element 2022 may be configured to combine the light combined by the second light combination element 2021 and the light emitted the second light source 1023 after the light is reflected and transmitted, respectively, to form the first incident beam incident onto the first light combination element 201. In some embodiments, the first light combination module 103 may include only one second light combination element 202. As shown in FIG. 3, the first light combination module 103 may include the first light combination element 201 and one second light combination element 202. The second light combination element 202 may be disposed between the first light combination element 201 and the at least one second light source 102. The second light combination element may reflect the second beam emitted by the at least one second light source 102 to be incident onto the first light combination element 201. The first light combination element 201 may combine the second beam emitted by the at least one second light source 102 with the first beam to form the combined light, and the combined light may be transmitted to the light guide module 800. In some embodiments, the first light combination module 103 may include only the first light combination element 201, but not the second light combination element. For example, as shown in FIG. 1, when the light source device 100 includes only one second light source 102, the first light combination module 103 may include only one first light combination element 102 configured to combine the beams emitted by the first light source 101 and the second light source 102 by transmitting and/or reflecting the beams respectively. The light source device 100 including a plurality of second light combination elements 202 may be found in the related descriptions of FIG. 4, FIG. 9, FIGs. 12-19, and FIG. 22 of the present disclosure.

In some embodiments, when the first light source 101 is a violet light source that emits a short-wave spectrum, a peak wavelength of the at least one second light source 102 may be longer than a peak wavelength of the violet light source, and the first light combination element 201 may be a short-wave dichroic mirror. In some embodiments, when the first light source 101 is a red light source that emits a narrowband spectrum, the peak wavelength of the at least one second light source 102 may be shorter than a peak wavelength of the red light source, and the first light combination element may be a long-wave dichroic mirror. The long-wave dichroic mirror or the short-wave dichroic mirror have fewer coating layers than a band-pass dichroic mirror and require shorter coating time. Therefore, when the first light combination element 201 and/or the at least one second light combination element 202 are the long-wave dichroic mirror or the short-wave dichroic mirror, a coating process of the dichroic mirror of the first light combination module 103 is simple and low in production cost. Meanwhile, short-wave or long-wave cutoff may be achieved using the long-wave pass dichroic mirror or the short-wave pass dichroic mirror, such that the color spectra from each light source (e.g., the first light source 101 and the at least one second light source 102 ) in the spectrum of the combined light are independent of each other, which simplifies the control strategy of the spectrum and the light flux, and better realizes the control of illumination light hue and light flux stability.

In some embodiments, when the first light source 101 is the violet light source that emits the short-wave spectrum and the count N of the at least one second light source 102 is at least two, peak wavelengths of the N second light sources 102 may be longer than the peak wavelength of the violet light source. A peak wavelength of a second beam emitted by the at least one second light source 102 and reflected by the at least one second light combination element 202 may be longer than a peak wavelength of a second beam emitted by the at least one second light source 102 and transmitted by the at least one second light combination element 202, and the beam is combined at the at least one second light combination element 202. In this case, the at least one second light combination element 202 may be the short-wave pass dichroic mirror.

In some embodiments, when the first light source 101 is the red light source that emits the narrowband spectrum and the count N of the at least one second light source 102 is at least two, peak wavelengths of the N second light sources 102 may be shorter than the peak wavelength of the red light source. A peak wavelength of a second beam emitted by the at least one second light source 102 and reflected by the at least one second light combination element 202 may be shorter than the peak wavelength of the second beam emitted by the at least one second light source 102 and transmitted by the at least one second light combination element 202, and the light may be combined at the at least one second light combination element 202. In this case, the at least one second light combination element 202 may be the long-wave pass dichroic mirror.

In some embodiments, when the first light source 101 is any one of a narrowband blue light source, a narrowband green light source, a narrowband amber light source, and a narrowband red light source, the first light combination element 201 or the at least one second light combination element 202 may be the long-wave pass dichroic mirror, the short-wave pass dichroic mirror, or the band-pass dichroic mirror.

In some embodiments, when the first light combination module 103 includes a plurality of light combination elements, an arrangement angle of each of the light combination elements may be set to save assembly space, thereby achieving assembly processability and a compact structure. For example, a first angle between each of the at least one second light combination element 202 and the first light combination element 201 may be less than a first preset angle. It should be understood that an angle between two light combination elements in the present disclosure refers to an acute angle or a right angle formed by planes where light combination surfaces of the two light combination elements are located. For example, when the first light combination element 201 and the at least one second light combination element 202 are dichroic mirrors, the first angle between each of the at least one second light combination element 202 and the first light combination element 201 may be an acute angle or a right angle formed by planes where light combination surfaces of the two dichroic mirrors are located. As another example, when the first light combination element 201 and the at least one second light combination element 202 are light combination prisms, the first angle between each of the at least one second light combination element 202 and the first light combination element 201 may be an acute angle or a right angle formed by planes where the light combination surfaces of the two light combination prisms are located. In some embodiments, the first preset angle may be 5°, 10°, 15°, 20°, etc. The first angle between each of the at least one second light combination element 202 and the first light combination element 201 may be less than the first preset angle, such that each light combination element may be parallel or tend to be parallel to each other in space, thereby avoiding mutual interference in the assembly space, and achieving the assembly processability and the compact structure.

In some embodiments, a second angle between the first light combination element 201 and an optical axis where the first light combination element is located may be greater than or equal to a second preset angle and less than or equal to a third preset angle. In some embodiments, a third angle between each of the at least one second light combination element 202 and an optical axis where the second light combination element 202 is located may be greater than or equal to a fourth preset angle and less than or equal to a fifth preset angle. It should be understood that the angle between the light combination element and the optical axis where the light combination element is located in the present disclosure refers to an acute angle between a light combination surface of the light combination element and the optical axis where the light combination element is located. As shown in FIG. 3, the second angle between the first light combination element 201 and the optical axis where the first light combination element 201 is located is an angle a, and the third angle between each of the at least one second light combination element 202 and the optical axis where the second light combination element 202 is located is an angle b. In some embodiments, the second preset angle, the third preset angle, the fourth preset angle, and/or the fifth preset angle may be 30°, 40°, 45°, 50°, 60°, etc. In some embodiments, the second preset angle, the third preset angle, the fourth preset angle, and the fifth preset angle may be four different angles. For example, the second preset angle may be 30°, the third preset angle may be 50°, the fourth preset angle may be 40°, and the fifth preset angle may be 50°. In some embodiments, the second preset angle and the fourth preset angle may be the same. For example, the second preset angle and the fourth preset angle may both be 40°. In some embodiments, the third preset angle and the fifth preset angle may be the same. For example, the third preset angle and the fifth preset angle may both be 50°.

In some embodiments, the first light combination element 201 and/or the at least one second light combination element 202 may be configured to combine the first beam or the second beam emitted by a corresponding first light source 101 and/or corresponding at least one second light source 102, and perform long-wave cutoff filtering, short-wave cutoff filtering, or narrowband filtering on the first beam or the second beam. For example, the dichroic mirror of the first light combination element 201 and/or the at least one second light combination element 202 may not only have a light combination function, but also perform long-wave cutoff filtering, short-wave cutoff filtering, or narrowband filtering on the light emitted by the corresponding light source, thereby simplifying the endoscope system 10 and reducing the cost.

In some embodiments, when the first light source 101 and/or the at least one second light source 102 include a blue light source, the corresponding first light combination element 201 or the corresponding at least one second light combination element 202 may perform long-wave cutoff on a blue band in the light emitted by the blue light source. For example, a transition zone wavelength of the dichroic mirror of the first light combination element 201 or the at least one second light combination element 202 may be in a range of 450 nm-470 nm, and the dichroic mirror may cut off a wavelength of which a spectrum is greater than 460 nm in the blue band. For example, the blue light source is a B-LED capable of emitting a blue band B light, with a peak wavelength being in a range of 430 nm-460 nm. For example, a peak wavelength of the blue light source may be in a range of 430 nm-450 nm, and a wavelength of the peak wavelength may be a narrow band with a bandwidth of about 20 nm or 30 nm. The difference in reflectivity between the surface or superficial blood vessels and the mucosa through a spectrum below 460 nm is large, which can improve the contrast between the surface blood vessels and the mucosa.

In some embodiments, when the first light source 101 and/or the at least one second light source 102 include a green light source (e.g., the green light source emits a green light by exciting a fluorescent material with a blue LED, and a spectrum of the light emitted by the green light source includes a green band spectrum and a blue excitation light), the dichroic mirror of the first light combination element 201 or the at least one second light combination element 202 may perform short-wave cutoff on the blue excitation light of the light emitted by the green light source, and the dichroic mirror may cut off a wavelength of a spectrum of the green light source less than 460 nm. For example, the green light source may include a fluorescent G_LED that emits green light by exciting a fluorescent material with a blue LED. The blue LED may have a blue excitation light with a peak wavelength being in a range of 410 nm-440 nm. The blue excitation light may excite a fluorescent material to produce a green light, and a small amount of blue excitation light may not be absorbed by the fluorescent material and may be directly transmitted. That is, the spectrum of the fluorescent G_LED may include a small amount of blue excitation light in addition to the green band spectrum. Compared with the LED that emits green light, the fluorescent G_LED may be easier to achieve high output light power.

In some embodiments, when the first light source 101 and/or the at least one second light source 102 include a violet light source, the dichroic mirror of the corresponding first light combination element 201 or the corresponding at least one second light combination element 202 may perform long-wave cutoff or narrowband filtering on a violet band. For example, the dichroic mirror may cut off a long-wave band with a wavelength higher than 410 nm in the violet band, or the dichroic mirror may perform ± 10 nm narrowband filtering centered at a wavelength of 405 nm in the violet band. For example, the first light source 101 and/or the at least one the second light source 102 may include a UV_LED that emits a UV light in a range from a violet band to a blue band, and the dichroic mirror may have a transition zone wavelength of about 400 nm-420 nm, which can cut off a wavelength of the spectrum greater than 410 nm of the UV_LED and perform cutoff filtering on a long-wave spectrum of the UV_LED. For example, the dichroic mirror has a bandpass characteristic of 405 ± 10 nm, and performs narrowband filtering of ±10 nm on the spectrum of UV_LED centered 405 nm to eliminate individual differences in LEDs (e.g., peak wavelength deviations of LEDs in different batches) and well limit the illumination spectrum to the high absorption band of hemoglobin to increase the contrast between the surface blood vessels and the mucosa.

In some embodiments, in order to achieve a higher optical efficiency of the combined light emitted by the light source device 100 in terms of design and adjustment, an optical path of the first light source 101 and/or the at least one second light source 102 may be designed. For example, only one light combination element (e.g., the first light combination element 201) may be disposed between the first light source 101 and the light guide module 800. In order to compensate for the insufficient light flux of a narrowband spectrum due to narrowband filtering, or the attenuation of the transmittance of a light guide beam in a short-wave band to achieve sufficient brightness of a specific spectrum when the endoscope system 10 is observed at close range and medium and long distances and make the image have a high contrast in the subsequent imaging process, a distance between the first light source 101 (e.g., the narrowband light source or the short-wave light source) and an entrance of the light guide module 800 may be less than or equal to a distance between each of the at least one second light source 102 and the entrance of the light guide module 800. It should be understood that the distance between the light source and the entrance refers to a total length of a path that the beam emitted by the light source passes through when reaching the entrance. As shown in FIG. 2, a distance L1 between the first light source 101 (e.g., the narrowband light source or the short-wave light source) and the entrance of the light guide module 800 may be less than a distance (L2+L3) between each of the at least one second light source 102 and the entrance of the light guide module 800. As shown in FIG. 3, the distance L1 between the first light source 101 (e.g., the narrowband light source or the short-wave light source) and the entrance of the light guide module 800 may be less than a distance (L2+L3+L4) between each of the at least one second light source 102 and the entrance of the light guide module 800. Setting the distance between the first light source 101 and the entrance of the light guide module 800 to the shortest distance can make the optical transmission efficiency of the first light source 101 high, which further improves the light flux of the first beam, and make it easy to assembly and adjust, thereby achieving higher optical transmission efficiency in terms of design and adjustment.

FIG. 4 is a schematic diagram illustrating an exemplary light source device according to some embodiments of the present disclosure. As shown in FIG. 4, the light source device 100 may include the first light source 101, three second light sources 102, and the first light combination module 103. The light guide module 800 may be connected with the light source device 100 and configured to output a combined light generated by the light source device 100. The three second light sources 102 may include a light source 1021, a light source 1022, and a light source 1023. The first light combination module 103 may include a first light combination element 201 and at least one second light combination element 202. As shown in FIG. 4, the at least one second light combination element 202 may include a light combination element 2021 and a light combination element 2022.

As shown in FIG. 4, a first beam related to the first light source 101 may be transformed through the first light combination element 201 to generate a first transmitted light. A second beam emitted by the light source 1021 of the three second light sources 102 may be transmitted through the at least one second light combination element 202 (e.g., transmitted through the light combination element 2021) and reflected (e.g., reflected through the light combination element 2022), and the second beam emitted by the light source 1022 of the three second light sources 102 may be transmitted through the at least one second light combination element 202 (e.g., transmitted through the light combination element 2022) to generate a first reflected light. The first light combination element 201 may combine the first reflected light and the first transmitted light to form the combined light. The combined light may enter the light guide module 800 and may be transmitted to a tissue. In some embodiments, the first light source 101 may include a narrowband light source or a short-wave light source.

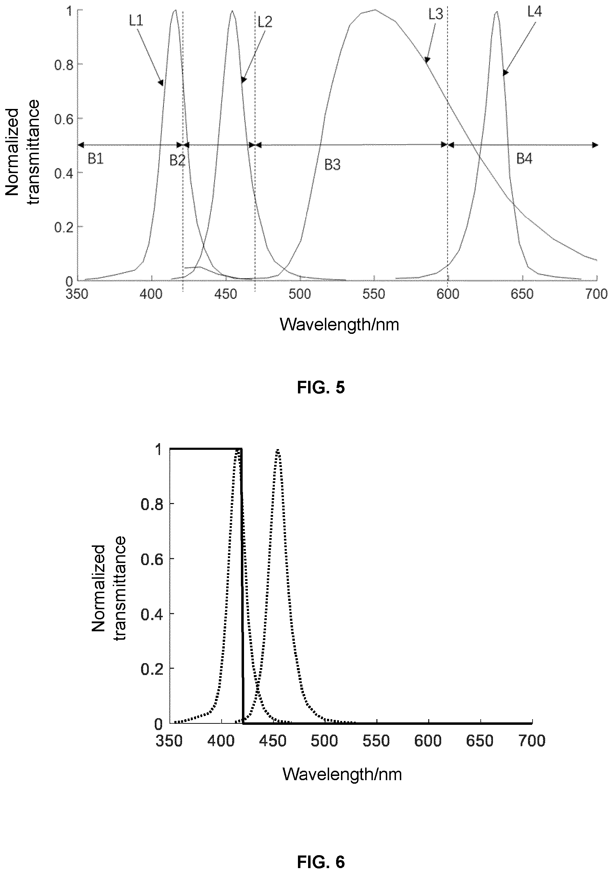

FIG. 5 is a schematic diagram illustrating exemplary spectrum curves of light sources according to some embodiments of present disclosure. L1, L2, L3, and L4 respectively represent spectrum curves of the first light source 101, the light source 1021, the light source 1022, and the light source 1023. Specifically, the first light source 101 may be a UV_LED that emits a UV light in a range from violet band to a blue band. The light source 1021, the light source 1022, and the light source 1023 may be a blue light source B-LED that emits a blue band B light, a green light source G-LED that emits a green band G light, and a red light source R-LED that emits a red band R light, respectively. A peak wavelength of the UV light emitted by the UV_LED may be less than a peak wavelength of the B light emitted by the B-LED.

In some embodiments, due to the strong absorption of hemoglobin to a 405 nm-415 nm spectrum, the first light source 101 may be a UV-LED with a peak wavelength being in a range of 405 nm-415 nm, and a wavelength of the first light source 101 may be in a narrowband range, and a bandwidth of the first light source 101 may be in a range of 15 nm-25 nm. For example, the bandwidth of the first light source 101 may be about 20 nm. Since the high scattering and strong absorption characteristics of the UV-LED, the UV-LED may be used to depict a morphology of blood vessels near a surface or superficial layer.

In some embodiments, the light source 1021 (e.g., the blue light source) of the three second light sources 102 may have a peak wavelength of 430 nm-460 nm. For example, a peak wavelength of the light source 1021 (e.g., blue light source) may be in a range of 430 nm-450 nm. In some embodiments, the surface blood vessels and the mucosa are distinguished in an observed image based on the difference in reflectivity between the surface blood vessels and the mucosa, and the wavelength of the light source 1021 is preferably a narrowband range, and the bandwidth is in a range of 15 nm-25 nm. For example, the bandwidth of the light source 1021 may be about 20 nm. In some embodiments, the light emitted by the UV_LED or the B_LED may not meet a bandwidth requirement. In this case, a narrowband filter may be disposed in a collimating optical path of the UV_LED or the B_LED to meet the bandwidth requirement.

In some embodiments, the light source 1022 (e.g., green light source) may have a peak wavelength of 510 nm-560 nm, and a bandwidth of the light source 1022 may be in a broadband range. For example, the bandwidth of the light source 1022 may be in a range of 90 nm-110 nm. For example, the bandwidth of the light source 1022 may be about 100 nm. In some embodiments, the light source 1022 may be a fluorescent G_LED that emits green light by exciting a fluorescent material with a blue LED. In some embodiments, the blue LED may have a blue excitation light with a peak wavelength of 410 nm-440 nm, and the fluorescent material may be excited by the blue excitation light to produce a green light. A small amount of blue excitation light may not be absorbed by the fluorescent material and may be directly transmitted. That is, the spectrum of the light source 1022 may include a small amount of blue excitation light in addition to the green band spectrum. Compared with the LED that emits green light, the fluorescent green LED may be easier to achieve high output light power.

In some embodiments, the light source 1023 (e.g., the red light source) may have a peak wavelength of 610 nm-640 nm, and a wavelength of the light source 1023 may be in a narrowband range. For example, the bandwidth of the light source 1023 may be in a range of 15 nm-25 nm. For example, the bandwidth of the light source 1023 may be about 20 nm.

FIG. 6 is a schematic diagram illustrating a transmittance spectrum of an exemplary first light combination element according to some embodiments of the present disclosure. Taking the first light combination element 201 as a dichroic mirror as an example, the first light combination element 201 shown in FIG. 6 has a short-wave pass characteristic with a transition zone wavelength of about 410 nm-430 nm, which can reflect a spectrum of the beam emitted by the light source 1021, the light source 1022, and the light source 1023 that is above 420 nm and transmit a spectrum of the beam emitted by the first light source 101 that is below 420 nm.

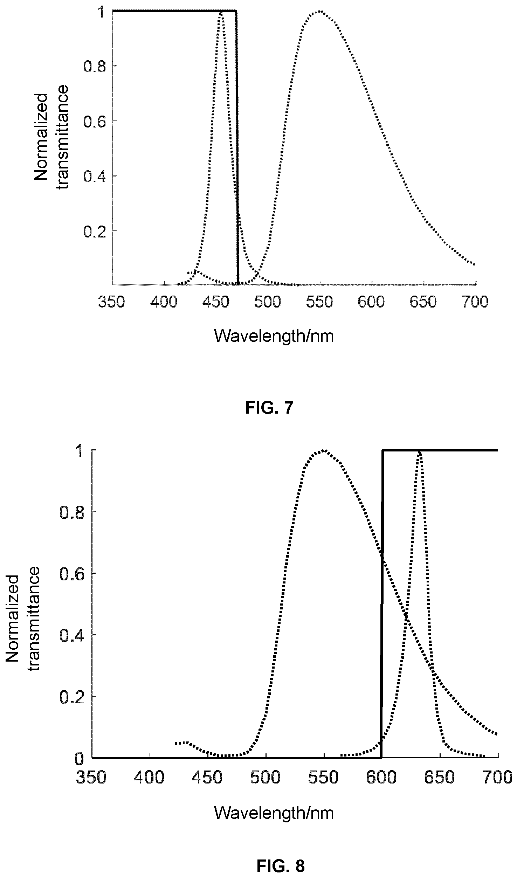

FIG. 7 is a schematic diagram illustrating a transmittance spectrum of an exemplary second light combination element (e.g., the light combination element 2021) according to some embodiments of the present disclosure. Taking the light combination element 2021 of the at least one second light combination element 202 as a dichroic mirror as an example, the light combination element 2021 shown in FIG. 7 has a short-wave pass characteristic with a transition zone wavelength of about 460 nm-480 nm, which can reflect a spectrum of the beam emitted by the light source 1022, that is above 470 nm and transmit a spectrum of the beam emitted by the light source 1021 that is below 470 nm.

FIG. 8 is a schematic diagram illustrating a transmittance spectrum of an exemplary second light combination element (e.g., the light combination element 2022) according to some embodiments of the present disclosure. Taking the light combination element 2022 of the at least one second light combination element 202 as a dichroic mirror as an example, the light combination element 2022 shown in FIG. 8 has a long-wave pass characteristic with a transition zone wavelength of about 590 nm-610 nm, which can reflect a spectrum of the beam emitted by the light source 1021 and the light source 1022 that is below 600 nm and transmit a spectrum of the beam emitted by the light source 1023 that is above 600 nm.

Referring to FIGs. 4-8, the first light combination element 201 and the at least one second light combination element 202 (e.g., the light combination element 2021 and the light combination element 2022) may be combined. The light combination element 2021 may transmit a beam emitted by the light source 1021 and reflect a beam emitted by the light source 1022 to obtain a combined beam A1. The light combination element 2022 may transmit a beam emitted by the light source 1023 and reflect the combined beam A1 to obtain a combined beam A2. The first light combination element 201 may transmit a beam emitted by the first light source 101, and reflect the combined light beam A2 to generate a combined light including spectrums output by the first light source 101, the light source 1021, the light source 1022, and the light source 1023, such that the combined light may be transmitted to the tissue by the light guide module 800. Combined with the transmittance of the first light combination element 201 and the at least one second light combination element 202 in FIGs. 5-8, each light source may finally obtain an independent spectrum after passing through the first light combination element 201 and each of the at least one second light combination element, which are a violet spectrum less than or equal to 410 nm, a blue spectrum greater than 410 nm and less than 470 nm, a green spectrum greater than 470 nm and less than 600 nm, and a red spectrum greater than 600 nm.

In some embodiments, the light source 101 (e.g., the violet light source), the light source 1021 (e.g., the blue light source), the light source 1022 (e.g., the green light source), and the light source 1023 (e.g., the red light source) may be mixed in a specific ratio to output ordinary white light illumination that meets a requirement, which can be used to generate a contour image of the surface mucosa. In some embodiments, special light illumination with the spectrum of the first light source 101 (e.g., the violet light source) or the light source 1021 (e.g., the blue light source) as the main spectrum can be used for surface and middle layer blood vessel emphasized observation. In some embodiments, the ordinary white light illumination and the special light illumination may be mixed. For example, the spectrum of the first light source 101 (e.g., the violet light source) or the light source 1021 (e.g., the blue light source) in the ordinary white light illumination may be appropriately increased, such that an image that considers the overall contour of the surface tissue and the emphasized observation of the blood vessels can be obtained.

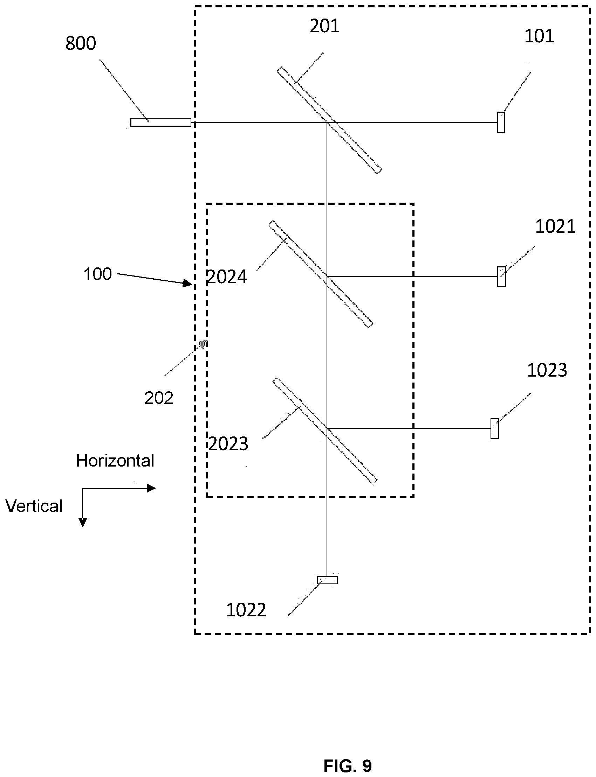

In some embodiments, in addition to the structure of the exemplary light source device provided in FIG. 4, a light combination order of the light sources may also be changed, and the light source device 100 may be adjusted in length and width directions to achieve an optimized spatial layout. FIG. 9 is a schematic structural diagram illustrating an exemplary light source device according to some embodiments of the present disclosure. As shown in FIG. 9, compared with the light source device shown in FIG. 4, the light source device 100 as a whole may be reduced in length in a horizontal direction and increased in length in a vertical direction. The length of the light source device 100 in the present disclosure refers to a maximum distance between two components of the light source device 100 in the corresponding direction.

As shown in FIG. 9, the light source device 100 may include the first light source 101, three second light sources 102, and the first light combination module 103. The light guide module 800 may be connected with the light source device 100 and configured to output a combined light generated by the light source device 100. The three second light sources 102 may include the light source 1021, the light source 1022, and the light source 1023. The first light combination module 103 may include the first light combination element 201 and at least one second light combination element 202. As shown in FIG. 9, the at least one second light combination element 202 may include a light combination element 2023 and a light combination element 2024.

As shown in FIG. 9, a first beam related the first light source 101 may be transformed through the first light combination element 201 to generate a first transmitted light. A second beam emitted by the light source 1021 of the three second light sources 102 may be reflected by the at least one second light combination element 202 (e.g., reflected by the light combination element 2024), a second beam emitted by the light source 1022 of the three second light sources 102 may be transmitted through the at least one second light combination element 202 (e.g., transmitted through the light combination element 2023 and the light combination element 2024, respectively), and a third beam emitted by the light source 1023 of the three second light sources 102 may be reflected by the at least one second light combination element 202 (e.g., reflected by the light combination element 202 ) to generate a first reflected light. The first light combination element 201 may combine the first reflected light and the first transmitted light to generate a combined light. The combined light may enter the light guide module 800 and may be transmitted to a tissue under test. In some embodiments, the first light source 101 may include a narrowband light source or a short-wave light source. For example, the light source 1021 may be a blue light source, the light source 1023 may be a red light source, the light source 1022 may be a green light source, and the first light source 101 may be a violet light source. As shown in FIG. 9, optical axes of the light source 1021 (e.g., the blue light source) and the light source 1023 (e.g., the red light source) may be parallel to an optical axis of an output light, and an optical axis of light source 1022 (e.g., the green light source) may be perpendicular to the optical axis of the output light.

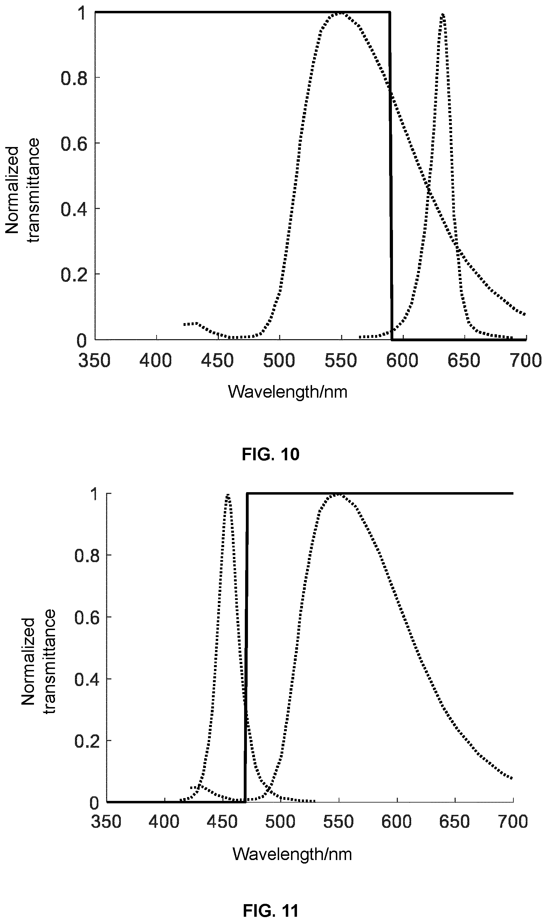

FIG. 10 is a schematic diagram illustrating a transmittance spectrum of an exemplary second light combination element (e.g., the light combination element 2023) according to some embodiments of the present disclosure. Taking the light combination element 2023 as a dichroic mirror as an example, the light combination element 2023 has a short-wave pass characteristic with a transition zone wavelength of about 590 nm-610 nm, which can transmit a spectrum of a beam emitted by the light source 1022 (e.g., the green light source) that is below 600 nm and reflect a spectrum of a beam emitted by the light source 1023 (e.g., the red light source) that is above 600 nm.

FIG. 11 is a schematic diagram illustrating a transmittance spectrum of an exemplary second light combination element (e.g., the light combination element 2024) according to some embodiments of the present disclosure. Taking the light combination element 2024 as a dichroic mirror as an example, the light combination element 2024 has a long-wave pass characteristic with a transition zone wavelength of about 460 nm-480 nm, which can transmit a spectrum of a beam emitted by the light source 1022 (e.g., the green light source) and the light source 1023 (e.g., the red light source) that is above 470 nm and reflect a spectrum of a beam emitted by the light source 1021 (e.g., the blue light source) that is below 470 nm.

Referring to the transmittance spectrum of the first light combination element 201 in FIG. 6, it can be seen that the first light combination element 201 has a short-wave pass characteristic with a transition zone wavelength of about 410 nm-430 nm. The first light combination element 201 may reflect a spectrum of a beam emitted by the light source 1021 (e.g., the blue light source), the light source 1022 (e.g., the green light source), and the light source 1023 (e.g., the red light source) that is above 420 nm and transmit a spectrum of a beam emitted by the first light source 101 that is below 420 nm. Referring to FIG. 6 and FIG. 9-11, it can be seen that each light source may finally obtain an independent spectrum after passing through the first light combination element 201, the light combination element 2023, and the second light combination element 2024 of the at least one second light combination element 202, which are respectively a violet light spectrum less than or equal to 420 nm, a blue light spectrum greater than 420 nm and less than 470 nm, a green light spectrum greater than 470 nm and less than 600 nm, and a red light spectrum greater than 600 nm.

In some embodiments, as shown in FIGs. 2-4 and FIG. 9, an optical axis of the first light source 101 may be coaxial with the optical axis of the output light of the light guide module 800. Since the first light source 101 is the narrowband light source or the short-wave light source, the coaxial arrangement of the optical axis of the first light source 101 and the optical axis of the output light of the light guide module 800 may form an optical path space for setting a filter, so as to realize the observation of the narrowband light or the short-wave light of the first light source 101.

In some embodiments, as shown in FIG. 9, optical axes of the light source 1021 and the light source 1023 of the at least one second light source 102 may be parallel to the optical axis of the output light of the light guide module 800, so as to facilitate setting a filter in an optical axis direction of the light source 1021 and/or the light source 1023 to perform narrowband filtering on the second beam emitted by the light source 1021 and/or the light source 1023, thereby realizing narrowband light observation of the light source 1021 and/or the light source 1023.

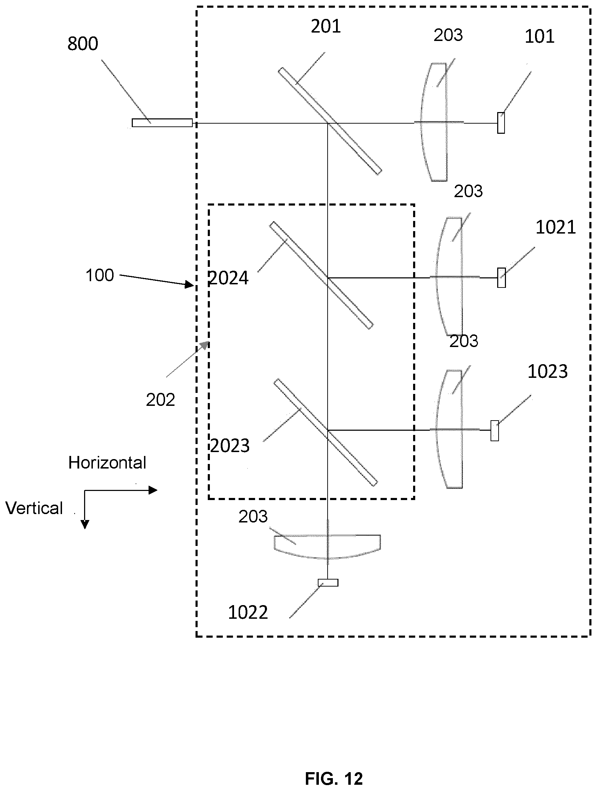

FIG. 12 is a schematic structural diagram illustrating an exemplary light source device according to some embodiments of the present disclosure. The difference between the light source device 100 shown in FIG. 12 and the light source device 100 shown in FIG. 9 is that the light combination module further includes one or more collimating lenses 203. In some embodiments, one of the one or more collimating lenses 203 may be disposed between the first light source 101 and the first light combination element 201, and configured to convert a first beam into a parallel beam or an approximately parallel beam to be incident onto the first light combination element 201. Optionally or additionally, one of the one or more collimating lenses 203 may be disposed between the at least one second light source 102 (e.g., the light source 1021, the light source 1022, or the light source 1023 ) and the corresponding at least one second light combination element 202 (e.g., the light combination element 2023 or the light combination element 2024), and configured to convert a second beam into a parallel beam or an approximately parallel beam to be incident onto the at least one second light combination element 202. For example, one of the one or more collimating lenses 203 may be disposed between the light source 1021 and the corresponding at least one second light combination element 202 (e.g., the light combination element 2024). As another example, one of the one or more collimating lenses 203 may be disposed between the light source 1022 and the corresponding at least one second light combination element 202 (e.g., the light combination element 2023). As another example, one of the one or more collimating lenses 203 may be disposed between the light source 1023 and the corresponding at least one second light combination element 202 (e.g., the light combination element 2023). It should be understood that the parallel beam or the approximately parallel beam in the present disclosure may be a beam of lights that are parallel or approximately parallel to each other (e.g., an angle between the lights may be within 5°).

In some embodiments, when the first light source 101 emits a divergent beam, one of the one or more collimating lenses 203 may be disposed between the first light source 101 and the first light combination element 201 to convert the first beam into the parallel beam to be incident onto the first light combination element 201, and one of the one or more collimating lenses 203 may be disposed between each of the at least one second light source 102 and the corresponding at least one second light combination element 202 to convert the second beam into the parallel beam to be incident onto the corresponding at least second light combination element 202. The optical path integration may be completed by using a dichroic mirror and other light combination elements.

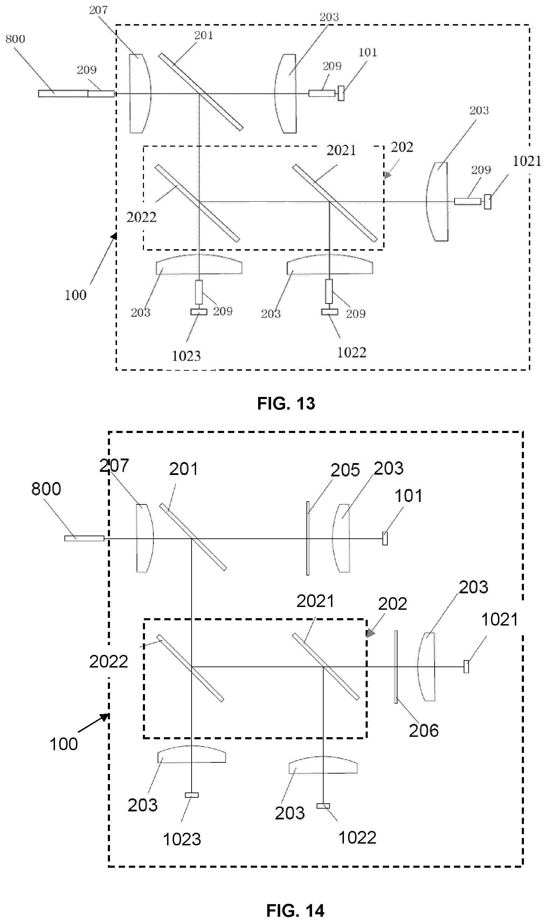

FIG. 13 is a schematic structural diagram illustrating an exemplary light source device according to some embodiments of the present disclosure. The difference between the light source device 100 shown in FIG. 13 and the light source device 100 shown in FIG. 12 is that the light source device 100 further includes one or more light guide components 209. In some embodiments, one of the one or more light guide components 209 may be disposed between the first light source 101 and the first light combination element 201, and configured to transmit a first beam to the first light combination element 201. Optionally or additionally, one of the one or more light guide components 209 may be disposed between the at least one second light source 102 (e.g., the light source 1021, the light source 1022, or the light source 1023) and the corresponding at least one second light combination element 202 (e.g., the light combination element 2023 or the light combination element 2024), and configured to transmit a second beam to the corresponding at least one second light combination element 202. Optionally or additionally, one of the one or more light guide components 209 may be disposed between the first light combination element 201 and the light guide module 800, and configured to transmit a combined light to the light guide module 800. It should be understood that the light combination element "corresponding" to the light source in the present disclosure refers to a light combination element through which the light emitted by the light source first passes. As shown in FIG. 13, the second light combination element 202 corresponding to the light source 1021 refers to the light combination element 2021; the second light combination element 202 corresponding to the light source 1022 refers to the light combination element 2021; the second light combination element 202 corresponding to the light source 1023 refers to the light combination element 2022.

In some embodiments, the light guide component 209 may be a light guide bundle or a light guide rod composed of a plurality of optical fibers, or a combination thereof. An entrance surface size of the light guide rod may be greater than or equal to an exit surface size of the light guide rod. When the entrance surface size of the light guide rod is greater than the exit surface size of the light guide rod, the light guide rod may be a tapered light guide rod.

In some embodiments, one of the one or more light guide components 209 may be disposed between the first light source 101 and the first light combination element 201, and between the at least one second light source 102 and the corresponding at least one second light combination element 202, respectively. As shown in FIG. 13, one of the one or more light guide components 209 may be disposed between the first light source 101 and one of the one or more collimating lenses 203 and/or between the at least one second light source 102 (e.g., the light source 1021, the light source 1022, or the light source 1023) and the corresponding collimating lens 203. For example, the first light source 101 or the at least one second light source 102 may be a laser light source, and the one or more light guide components 209 may be tapered light guide rods which amplify an divergence angle of the laser light source.

In some embodiments, one of the one or more light guide components 209 may also be disposed between the first light combination element 201 and the light guide module 800 to transmit the combined light to the light guide module 800. As shown in FIG. 11, one of the one or more light guide components 209 may be disposed between a focusing lens 207 and the light guide module 800 such that a non-uniformly distributed beam incident into the light guide component 209 may be homogenized to obtain a uniformly distributed emergent light to enter the light guide module 800.

In some embodiments, the first light source 101 and the at least one second light source 102 may be changed from a fixed position to other optimized spatial positions according to a free bending characteristic of the light guide bundle (e.g., light guide optical fibers), so as to facilitate a better heat dissipation effect for the first light source 101 and the at least one second light source 102. Furthermore, the light guide rod or the tapered light guide rod may have a light homogenizing effect, and the tapered light guide rod may transform a light emitting area and a light emitting angle of the beam, so as to output the light emitted by the first light source to the subsequent first light combination element 201 with high optical efficiency, or make the combined light output by the first light combination element 201 be incident into the subsequent light guiding module 800 with higher optical efficiency; or the one or more light guide components 209 may be combined with the light guide bundle (e.g., the light guide optical fibers) or the light guide rod to consider the above effects.

FIG. 14 is a schematic structural diagram illustrating an exemplary light source device according to some embodiments of the present disclosure. The difference between the light source device 100 shown in FIG. 14 and the light source device 100 shown in FIG. 12 is that the light combination module further includes a first filter 205 and/or one or more second filters 206. In some embodiments, the first filter 205 may be disposed between the first light source 101 and the first light combination element 201, and configured to transmit a beam of a first target waveband of a first beam (or light emitted by the first light source). For example, the first filter 205 may be disposed between one of the one or more collimating lenses 203 and the first light combination element 201. Optionally or additionally, one of the one or more second filters 206 may be disposed between the at least one second light source 102 (e.g., the light source 1021, the light source 1022, or the light source 1023) and the corresponding at least one second light combination element 202 (e.g., the light combination element 2021 or the light combination element 2022), and configured to transmit a beam of a second target waveband of a second beam. For example, one of the one or more second filters 206 may be disposed between a second collimating lens 203 corresponding to the at least one second light source 102 (e.g., the light source 1021, the light source 1022, or the light source 1023) and the corresponding at least one second light combination element 202 (e.g., the light combination element 2021 or the light combination element 2022). In some embodiments, the light source device 100 may further include a filter switch-in and switch-out module. The filter switch-in and switch-out module may include a filter configured to switch between a filter switch-in mode and a filter switch-out mode. The filter switch-in mode refers to the light being emitted after passing through the filter, and the filter switch-out mode refers to the light being emitted directly without passing through the filter. Bandwidths of a light output at a position of the filter in the filter switch-in mode and the filter switch-out mode may be different. For example, in the filter switch-in mode, the light output at the position of the filter may be a light after narrowband filtering; in the filter switch-out mode, the light output at the position of the filter may be a light without narrowband filtering.

In some embodiments, a wavelength of the first filter 205 disposed between the first light source 101 (e.g., the violet light source) and the first light combination element 201 may be a narrowband filter with a bandwidth of about 20 nm, and the first target waveband may be in a range of 390 nm-410 nm, which is used to depict the morphology of blood vessels near the surface or the superficial surface.

In some embodiments, the at least one second light source 102 (e.g., the blue light source) may have a peak wavelength of 430 nm-460 nm. Therefore, one of the one or more second filters 206 with a narrow bandwidth of about 20 nm may be disposed between the at least one second light source 102 (e.g., the blue light source) and the at least one second light combination element 202 to obtain a second target waveband of 430 nm -450 nm, and the surface blood vessels and the mucosa may be distinguished in an observed image based on the difference in reflectivity between the surface blood vessels and the mucosa. In some embodiments, the first target waveband and/or the second target waveband may be set according to the actual needs of observing the tissue.

In some embodiments, the at least one second light source 102 (e.g., the green light source) may preferably have a peak wavelength of 510 nm-560 nm, and a bandwidth may be in a range of 90 nm-110 nm. For example, the bandwidth of the at least one second light source 102 (e.g., the green light source) may be about 100 nm. In some embodiments, the at least one second light source 102 (e.g., the green light source) may be a light source that emits a green light by exciting a fluorescent material with a blue LED. For example, the blue LED may have a blue excitation light with a peak wavelength of 410 nm-440 nm, and the blue excitation light may excite the fluorescent material to produce the green light. A small amount of blue excitation light may not be absorbed by the fluorescent material and may be directly transmitted. That is, a spectrum of the at least one second light source 102 (e.g., the green light source) may contain a small amount of blue excitation light in addition to the green spectrum. Compared with an LED that emits the green light, the fluorescent green LED may be easier to achieve high output light power.

In some embodiments, as shown in FIG. 14, the light combination module may further include the focusing lens 207. In some embodiments, the focusing lens 207 may be disposed between the first light combination element 201 and the light guide module 800, and configured to focus the combined light to obtain a focused beam coupled into the light guiding module 800. In some embodiments, the focusing lens 207 may converge the combined light to form the focused beam with a certain aperture angle at a light exit, and the focused beam may be coupled into the light guide module 800.

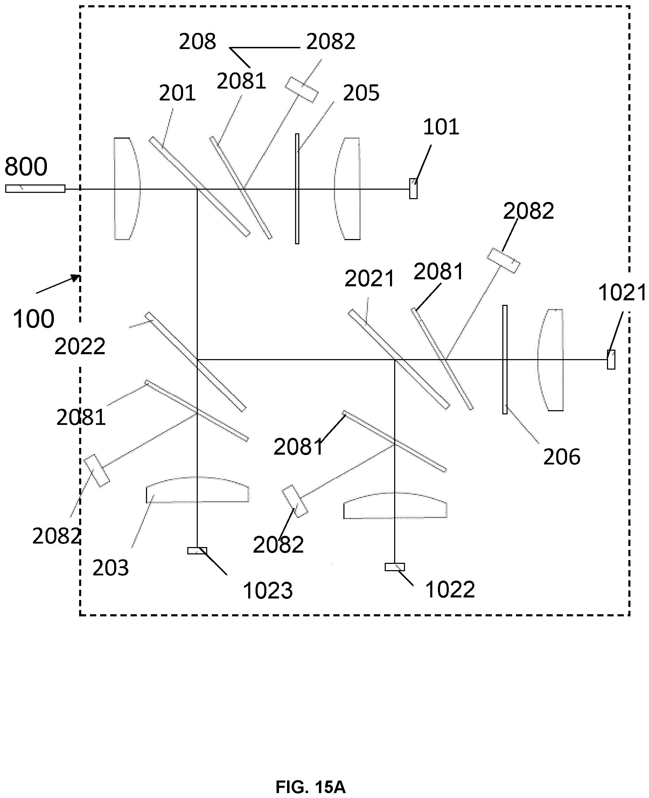

FIG. 15A is a schematic structural diagram illustrating an exemplary light source device according to some embodiments of the present application. The difference between the light source device 100 shown in FIG. 15A and the light source device 100 shown in FIG. 14 is that the light source device 100 further includes at least one light flux measurement module 208. In some embodiments, each of the at least one light flux measurement module 208 may include a beam splitter 2081 and a photodetector 2082 corresponding to the beam splitter 2081. In some embodiments, the beam splitter 2081 may be disposed between the first light source 101 and the first light combination element 201, and configured to split and reflect a first beam to obtain a third beam. The third beam may be incident into the photodetector 2082 corresponding to the beam splitter 2081. Optionally or additionally, the beam splitter 2081 may be disposed between each of the at least one second light source 102 (e.g., the light source 1021, the light source 1022, or the light source 1023) and the at least one second light combination element 202 (e.g., the light combination element 2021 or the light combination element 2022 ) corresponding to each of the at least one second light source 102, and configured to split and reflect a second beam to obtain a fourth beam. The fourth beam may be incident into the photodetector 2082 corresponding to the beam splitter 2081. The photodetector 2082 may be configured to detect a light flux of the third beam and/or the fourth beam incident into the photodetector 2082.

In some embodiments, the beam splitter 2081 may be disposed between the first light source 101 and the first light combination element 201, and may present a certain angle with an optical axis where the beam splitter 2081 is located. If other components are disposed between the first light source 101 and the first light combination element 201, such as one of the one or more collimating lens 203, the first filter 205, etc., the beam splitter 2081 may be disposed between the first filter 205 and the first light combination element 201 to split the first beam to obtain the third beam. The third beam may be incident into the photodetector 2082 corresponding to the beam splitter 2081. The photodetector 2082 may detect the third beam incident onto a photosensitive surface of the photodetector 2082 to obtain a detection light flux of the third beam.

In some embodiments, the at least one light flux measurement module may be provided in combination with a control portion to realize real-time feedback control of the light flux output by each light source. Meanwhile, in combination with a long-wave pass dichroic mirror or a short-wave pass dichroic mirror, the output spectra of each color are independent of each other. The filter is provided in a light flux measurement optical path to cut off a non-effective output spectrum portion, so as to realize a measurement spectrum that is consistent with or similar to an output spectrum of each light source in the output light. The detection light flux of each light source and the light flux output is strongly correlated, which ensures the accuracy of the light flux detection, thereby maintaining the stability of the illumination light color hue and the light flux, and simplifying the light flux control strategy.

In some embodiments, in order to avoid interference in the assembly space and further improve the assembly processability and the structural compactness, an angle between a reflection surface of the beam splitter 2081 and an optical axis where the beam splitter 2081 is located may be in a range of 50°-70°. For example, the beam splitter 2081 and the corresponding first light combination element 201 may tend to be parallel. In some embodiments, in order to avoid interference in the assembly space and further improve the assembly processability and the structural compactness, an angle between the beam splitter 2081 and the corresponding first light combination element 201 or the corresponding at least one second light combination element 202 may be less than a sixth preset angle. The sixth preset angle may be 15°. For example, an angle between the first light combination element 201 and the optical axis where the first light combination element 201 is located may be 45°, and the angle between the beam splitter 2081 and the optical axis where the beam splitter 2081 is located may be 60°, and an angle between the first light combination element 201 and the beam splitter 2081 may be 15°, which matches the spatial setting of the photodetector 2082 to obtain an optimal spatial layout, and further improves the assembly processability and the structural compactness. It should be noted that when the beam splitter 2081 performs beam splitting, a splitting ratio of the beam splitter 2081 is ≤ 10%. In this way, sufficient detection light flux is obtained, and reduction of the light flux caused by too much reduction in the effective illumination light entering the subsequent optical path for integration is avoided.

In some embodiments, the specific position of the beam splitter 2081 disposed between the at least one second light source 102 and the corresponding at least one second light combination element 202 may be found in the position of the beam splitter 2081 disposed between the first light source 101 and the first light combination element 201, and may be configured to split and reflect the second beam to obtain a fourth beam. The fourth beam may be incident into the photodetector 2082 corresponding to the beam splitter 2081. The photodetector 2082 may be configured to obtain the detection light flux of the fourth beam.

In some embodiments, the beam splitter 2081 may be a beam splitter plate or other optical elements with a beam-splitting property. The photodetector 2082 may be a photodiode (PD) or may be other types of light flux measurement modules, which is not limited in the embodiments of the present disclosure.

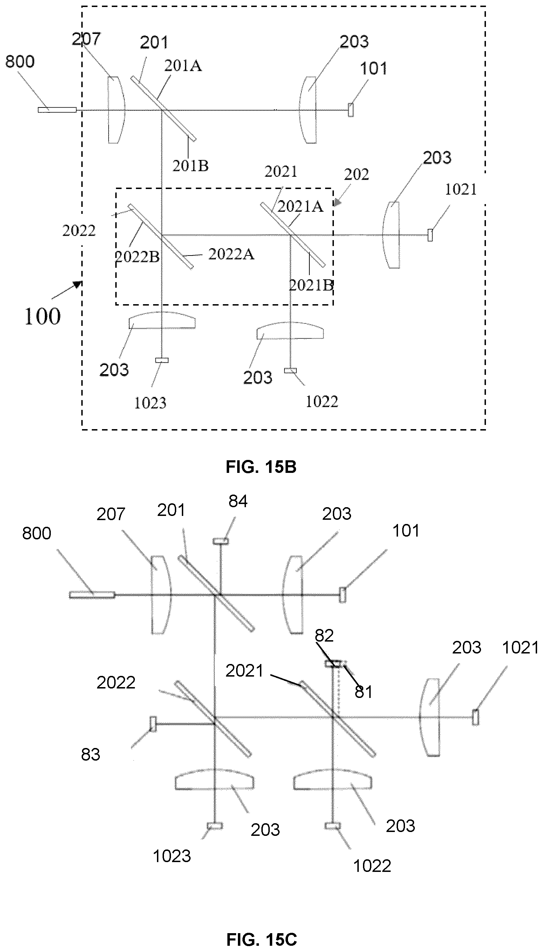

FIG. 15B is a schematic structural diagram illustrating an exemplary light source device according to some embodiments of the present application. FIG. 15C is a schematic structural diagram illustrating an exemplary light source device according to some embodiments of the present application. The light source device 100 shown in FIG. 15B and FIG. 15C has the same optical path and optical devices as the light source device 100 shown in FIG. 14, except that a filter is not included. It should be understood that the optical devices shown in the figure are only examples and do not limit the optical devices included in the light source device. The light source device may include a combination of a plurality of optical devices in different figures. Referring to FIG. 15B and FIG. 15C, the first light combination element 201, and the light combination element 2021 and the light combination element 2022 of the at least one second light combination element 202 may be respectively a first dichroic mirror, a second dichroic mirror, and a third dichroic mirror. Light emitted by the light source 1021 of the at least one second light source 102 may enter a first photodetector 81 after being split by a second optical surface 2021A on the third dichroic mirror. Light emitted by the light source 1023 of the at least one second light source 102 may enter a third photodetector 83 after being split by a second optical surface 2022 B on the second dichroic mirror. Light emitted by the first light source 101 may enter a fourth photodetector 84 after being split by a second optical surface 201A on the first dichroic mirror. The light emitted by the light source 1022 of the at least one second light source 102 may enter a second photodetector 82 after being transmitted through a first optical surface 2021B on the third dichroic mirror. The light emitted by the light source 1023 of the at least one second light source 102 may be transmitted through a first optical surface 2022A on the second dichroic mirror, and then transmitted through a first optical surface 201B on the first dichroic mirror to obtain a detection beam, and enter the corresponding third photodetector 83 for measurement. The first photodetector 81, the second photodetector 82, the third photodetector 83, and the fourth photodetector 84 may be photodiodes (PDs), which may also be other types of light flux measurement modules.

In some embodiments, the light source 1021 of the at least one second light source 102 may be a UV_LED that emits a UV light in a range from a violet band to a blue band. The light source 1022 of the at least one second light source 102 may be a B_LED that emits a blue band B light. The light source 1023 of the at least one second light source 102 may be a G_LED that emits a green band G light. The first light source 101 may be an R_LED that emits a red band R light. The UV_LED may preferably have a peak wavelength of 405 nm-415 nm according to strong absorption of hemoglobin to a 405 nm-415 nm spectrum, a wavelength of the UV_LED may be preferably in a narrowband range, and a bandwidth of the UV_LED may be about 20 nm. The UV_LED may be used to depict the morphology of blood vessels near the surface or the superficial surface according to the high scattering and strong absorption characteristics of the UV_LED. The B_LED may preferably have a peak wavelength of 430 nm-460 nm, and a peak wavelength of the B_LED may be preferably 430 nm-450 nm. The surface blood vessels and the mucosa may be distinguished in an observed image based on the difference in reflectivity between the surface blood vessels and the mucosa, a wavelength of the B_LED may be preferably in a narrowband range, and a bandwidth of the B_LED may be about 20nm. The G_LED may preferably have a peak wavelength of 510 nm-560 nm, and a bandwidth of the G_LED may be in a broadband range, such as a bandwidth of being about 100nm, and the G_LED may be a fluorescent LED. The R_LED may preferably have a peak wavelength of 600 nm-640 nm, a wavelength of the R_LED may preferably in a narrowband range, and a bandwidth of the R_LED may be about 20 nm.

In some embodiments, the light source 1023 of the at least one second light source 102 may be a blue LED that excites a fluorescent material to emit a green light. For example, the light source 1023 may be a fluorescent G_LED. The blue LED may have a blue excitation light with a peak wavelength being in a range of 410 nm-440 nm. The blue excitation light may excite the fluorescent material to produce the green light. A small amount of blue excitation light may not be absorbed by the fluorescent material and may be directly transmitted. Therefore, a spectrum of the light source 1023 of the at least one second light source 102 may include a small amount of blue excitation light in addition to a green spectrum. Compared with an LED that emits the green light, the fluorescent green LED may be easier to achieve high output light power.

In some embodiments, the first optical surface 2022 A on the second dichroic mirror may implement cutoff filtering on a short-waveband blue laser light in the light emitted by the G_LED to prevent the blue excitation light from entering a subsequent optical path. The spectral bands of the G_LED and the B_LED in the output illumination light hardly overlap with each other. By independently adjusting the proportions of the spectral components of each color, the control strategy of the spectrum and the light flux is simplified, thereby achieving high-precision control of the illumination light color hue and light flux stability.

In some embodiments, spectrum curves of the light source 1021 of the at least one second light source 102, the light source 1022 of the at least one second light source 102, the light source 1023 of the at least one second light source 102, and the first light source 101 are shown in FIG. 5. A spectrum L1 corresponds to a UV_LED ultraviolet light spectrum, a spectrum L2 corresponds to a B_LED blue light spectrum, a spectrum L3 corresponds to a hybrid light spectrum of G_LED blue excitation light and (fluorescent) green light, and a spectrum L4 corresponds to an R_LED red light spectrum.

Referring to FIG. 5 and FIG. 15B, the first optical surface 2021B on the third dichroic mirror may have a short-wave pass characteristic with a transition zone wavelength of about 410 nm-430 nm, which may be configured to transmit the light emitted by the UV_LED that is below 420 nm and reflect the light emitted by the B_LED that is above 420 nm, thereby completing the optical path integration of the violet light emitted by the UV_LED and the blue light emitted by the B_LED. Referring to FIG. 10 and FIG. 15B, the first optical surface 2022 A on the second dichroic mirror may have a long-wave pass characteristic with a transition zone wavelength of about 460 nm-480 nm, which may be configured to reflect the light emitted by the UV_LED and the B_LED that is below 470 nm and transmit the light emitted by the G_LED that is above 470 nm, thereby completing the optical path integration of the violet light emitted by the UV_LED, the blue light emitted by the B_LED, and the green light emitted by the G_LED.

Referring to FIG. 8 and FIG. 15B, the first optical surface 201B on the first dichroic mirror may have a long-wave pass characteristic with a transition zone wavelength of about 590 nm-610 nm, which may be configured to reflect the light emitted by the UV_LED, the B_LED and the G_LED that is below 600 nm and transmit the light emitted by the R_LED that is above 600 nm, thereby completing the optical path integration of the violet light emitted by the UV_LED, the blue light emitted by the B_LED, the green light emitted by the G_LED, and the red light emitted by the R_LED to output a combined light.

In some embodiments, the UV_LED may be spectrally separated from the B_LED through the long-wave cutoff of the first optical surface 2021B on the third dichroic mirror to achieve an independent spectrum B1 (≤420nm); the B_LED may be spectrally separated through the short-wave cutoff of the first optical surface 2021B on the third dichroic mirror and the long-wave cutoff of the first optical surface 2022A on the second dichroic mirror to achieve an independent spectrum B2 (420 nm-470 nm); the G_LED may be spectrally separated through the short-wave cutoff of the first optical surface 2022A on the second dichroic mirror and the long-wave cutoff of the first optical surface 201B on the first dichroic mirror to achieve an independent spectrum B3 (470 nm-600 nm); the R_LED may be spectrally separated through the short-wave cutoff (600 nm) of the first optical surface 2021B on the third dichroic mirror to achieve an independent spectrum B4 (≥600nm). The short-wave cutoff and the long-wave cutoff in the present disclosure are a short-wave end and a long-wave end of a light-emitting band of each LED relative to a specific light-emitting band.

The first dichroic mirror, the second dichroic mirror ,and the third dichroic mirror in the present disclosure can realize independent spectra B1-B4 of the UV_LED, the B_LED, the G_LED, and the R_LED spectrum curves while outputting the combined light after optical path integration of the violet light emitted by the UV_LED, the blue light emitted by the B_LED, the green light emitted by the G_LED, and the red light emitted by the R_LED. As shown in FIG. 5, the spectral bands of the UV_LED, the B_LED, the G_LED and the R_LED components in the illumination light outputted in the present disclosure almost do not overlap with each other. By independently adjusting the proportion of the spectral components of each color, the control strategy of the spectrum and light flux is simplified, and high-precision control of the illumination light color hue and light flux stability is achieved.

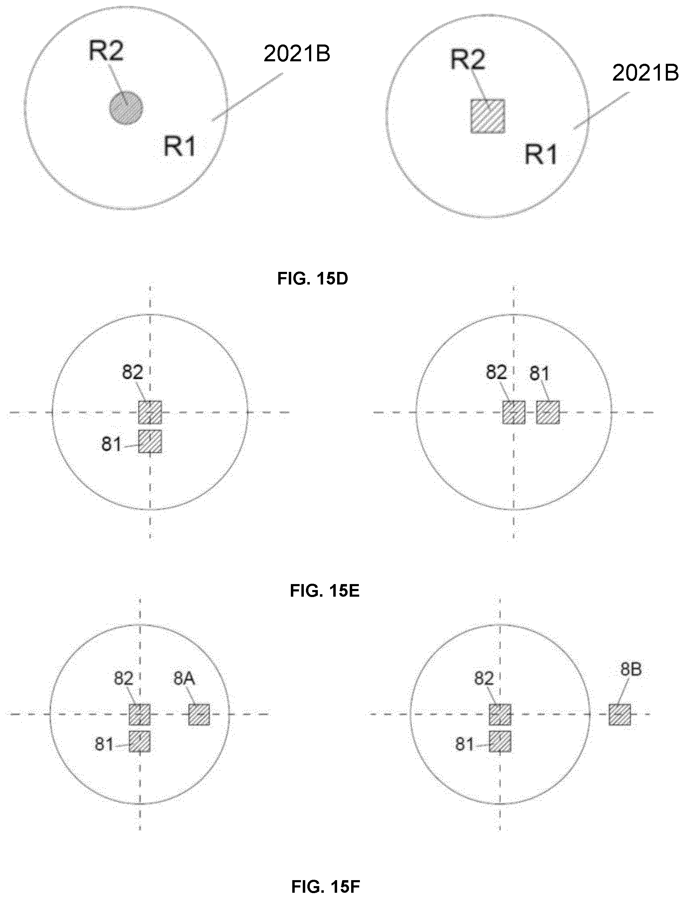

In some embodiments, a first optical zone R1 and a second optical zone R2 may be disposed on a first optical surface of at least one of the first dichroic mirror, the second dichroic mirror, and the third dichroic mirror. The third dichroic mirror is used for illustration below. FIG. 15D is a schematic diagram illustrating a coating of a first optical surface of an exemplary third dichroic mirror according to some embodiments of the present disclosure. FIG. 15E is a schematic diagram illustrating positions of an exemplary first photodetector and an exemplary second photodetector according to some embodiments of the present disclosure. FIG. 15F is a schematic diagram illustrating a position of an exemplary background light detector relative to a first photodetector and a second photodetector according to some embodiments of the present disclosure.

In some embodiments, the first optical zone R1 may occupy an area greater than or equal to 90% of an area of the first optical surface 2021B, and the second optical zone R2 may occupy an area less than or equal to 10% of the area of the first optical surface 2021B. The second optical zone R2 may be configured to transmit the light emitted by the corresponding light source such that the light enters the corresponding light flux measurement module. For example, in order to facilitate the detection of the light flux in the light source 1022(e.g., the B_LED) of the at least one second light source 102, the first optical surface 2021B on the third dichroic mirror may have a partition coating characteristic. Specifically, as shown in the left and right figures in FIG. 15D, the present disclosure is provided with the first optical zone R1 and the second optical zone R2 on the first optical surface 2021B on the third dichroic mirror. The first optical zone R1 and the second optical zone R2 may have different coating characteristics. The first optical zone R1 may occupy an area greater than or equal to 90% of the area of the first optical surface 2021B, and the second optical zone R2 may occupy an area less than or equal to 10% of the area of the first optical surface 2021B.

The first optical zone R1 may be configured to transmit a beam with a wavelength lower than 420 nm emitted by the light source 1021 of the at least one second light source 102 and reflect a beam with a wavelength greater than 420 nm emitted by the light source 1022 of the at least one second light source 102 to generate a combined light. The second optical zone R2 may be configured to transmit the light emitted by the light source 1022 of the at least one second light source 102 such that the light enters the second photodetector 82.

In some embodiments, the first optical zone R1 may have a dichroic filter film, and the second optical zone R2 may not be coated. In some embodiments, a beam splitting film may be provided on the second optical zone R2 to mainly transmit a blue light emitted by the B_LED. In some embodiments, an antireflection film with an antireflection property may be provided on the second optical zone R2, so as to transmit the light emitted by the B_LED through the second optical zone R2 to the corresponding second photodetector 82.

In some embodiments, as shown in FIG. 15D , the second optical zone R2 may be a square shape or a circular shape, and a size and a shape of the second optical zone R2 may be designed to match a size of a photosensitive surface of the second photodetector 82. That is, a transmitted beam of the light emitted by the B_LED passing through the second optical zone R2 may enter the photosensitive surface of the second photodetector 82 as a detection light, and a size of the detection light may be greater than or approximately equal to the size of the photosensitive surface of the second photodetector 82.

In some embodiments, when the second optical zone R2 is not coated, according to the Fresnel reflection characteristic of an optical material, if BK7 optical glass is used as a base material of the third dichroic mirror, the second optical zone R2 may have a transmittance of nearly 90%, which can achieve transmission splitting of the light emitted by the B_LED, and has the characteristic of simplified process.

In some embodiments, a photosensitive surface of at least one light flux measurement module corresponding to a light transmitted through the second optical zone R2 may be directly opposite to a direction of a detection optical axis of the dichroic mirror transmitted through the second optical zone. For example, referring to FIG. 15B, FIG. 15C, and FIG. 15D, a direction of the light transmitted from the second optical zone R2 to the photosensitive surface of the second photodetector 82 may be directly opposite to a direction of an optical axis (e.g., the detection optical axis) of a detection beam transmitted from the light source 1022 of the at least one second light source 102 through the second optical zone R2 of the dichroic mirror 2021. Meanwhile, the detection beam of the light emitted by the light source 1022 of the at least one second light source 102 after being transmitted through the second optical zone R2 of the first optical surface 2021B on the third dichroic mirror may be directly irradiated to the photosensitive surface on the second photodetector 82, such that the second photodetector 82 can optimally receive the corresponding detection light.

In some embodiments, a size of the beam transmitted to the corresponding light flux measurement module through the second optical zone R2 may be greater than a size of the photosensitive surface, and the detection beam emitted by the light source 1022 of the at least one second light source 102 may completely cover the photosensitive surface on the second photodetector 82. For example, referring to FIG. 15B, FIG. 15C, and FIG. 15D, a size of a beam emitted by the light source 1022 (e.g., the B_LED) of the at least one second light source 102 transmitted to the second photodetector 82 through the second optical zone R2 may be greater than a size of the photosensitive surface of the second photodetector 82. Therefore, the detection light of the B_LED incident into the second photodetector 82 may have a certain margin to cover the photosensitive surface of the second photodetector 82, such that the second photodetector 82 is insensitive to a mounting position, thereby ensuring the reliability of the system and controlling the production cost.

**In** some embodiments, a photosensitive surface of the at least one light flux measurement module corresponding to the light reflected by the second optical surface may be perpendicular to the direction of the detection optical axis of the dichroic mirror reflected by the second optical surface. For example, as shown in FIG. 15B and FIG. 15C, light reflected in a vertical direction by the light source 1021 of the at least one second light source 102 through the second optical surface 2021A on the third dichroic mirror may be in the direction of the detection optical axis, and the light reflected in the vertical direction through the second optical surface 2021A on the third dichroic mirror may be perpendicular to the photosensitive surface of the corresponding first photodetector 81. A photosensitive surface of the third photodetector 83 may be perpendicular to the detection optical axis reflected by the second optical surface 2022B on the corresponding second dichroic mirror. A photosensitive surface of the fourth photodetector 84 may be perpendicular to the detection optical axis reflected by the second optical surface 201A on the corresponding first dichroic mirror.

Since the photosensitive surfaces of the first photodetector 81, the third photodetector 83 and the fourth photodetector 84 are perpendicular to the detection optical axis reflected by the second optical surface 2021A on the third dichroic mirror, the second optical surface 2022B on the second dichroic mirror, and the second optical surface 201A on the first dichroic mirror, respectively, the first photodetector 81, the third photodetector 83 and the fourth photodetector 84 can optimally receive the corresponding detection light.

**In** some embodiments, a size of a beam reflected by the second optical surface to the photosensitive surface of the corresponding light flux measurement module may be much less than a size of a beam of a detection light emitted by the corresponding light source. For example, the size of the photosensitive surface of the first photodetector 81 may be much less than the size of the beam of the detection light emitted by the light source 1021 of the at least one second light source 102, the size of the photosensitive surface of the third photodetector 83 may be much less than the size of the beam of the detection light emitted by the light source 1023 of the at least one second light source 102, and the size of the photosensitive surface of the fourth photodetector 84 may be much less than the size of the beam of the detection light emitted by the first light source 101. Specifically, the size of the beam of the detection light reflected by the light source 1021 of the at least one second light source 102, the light source 1022 of the at least one second light source 102, the light source 1023 of the at least one second light source 102, and the first light source 101 through the corresponding second optical surface may be much greater than the size of the photosensitive surface of the corresponding first photodetector 81, the second photodetector 82, the third photodetector 83, and the fourth photodetector 84, respectively, such that the at least one light flux measurement module is insensitive to the mounting position, the reliability of the overall device is improved, and the production cost is reduced.

**In** some embodiments, in order to avoid the spatial position interference between the first photodetector 81 and the second photodetector 82, during the design, as shown in FIG. 15E, the first photodetector 81 for receiving the light reflected from the second optical surface may be appropriately offset in spatial position to avoid the space where the second photodetector 82 for receiving the light transmitted from the first optical surface is located. For example, the first photodetector 81 and the second photodetector 82 may be offset vertically, or offset in a left-right direction in the space of the detection optical path. As shown in FIG. 15E, a circle diameter shown in the figure is a beam diameter of the detection optical axis, and the detection beam is a beam after an approximately collimated beam is reflected by the second optical surface of the dichroic mirror. The first photodetector 81 and the second photodetector 82 may be arranged side by side vertically or in the left-right direction to optimally receive the detection light emitted by the light source 1021 of the at least one second light source 102 and the light source 1022 of the at least one the second light source 102. After the first photodetector 81 is offset, the beam of the detection light emitted by the light source 1022 of the at least one second light source 102 still fully covers the photosensitive surface of the second photodetector 82. In this case, the received light flux is not less than 90% of an original light flux.

It should be understood that the light source device 100 may include only one dichroic mirror or a plurality of dichroic mirrors. When the light source device 100 includes the plurality of dichroic mirrors, a beam splitting film may be respectively provided on the second optical surfaces of the plurality of dichroic mirrors. The beam splitting film may be configured to split the light emitted by the corresponding light source. Each beam splitting film may be configured to split the light of a different wavelength. A beam splitting wavelength of each beam splitting film may be determined by a wavelength of the light.

In some embodiments, the second optical surface 201A on the first dichroic mirror, the second optical surface 2022B on the second dichroic mirror, and the second optical surface 2021A on the third dichroic mirror may have a splitting characteristic, which is supplemented by a reflection characteristic and dominated by a transmission characteristic (e.g., reflect a small amount of light and transmit most of light) to achieve reflective splitting. When the light after reflective splitting is irradiated into the corresponding photodetector, the detection of the corresponding LED light flux can be achieved.

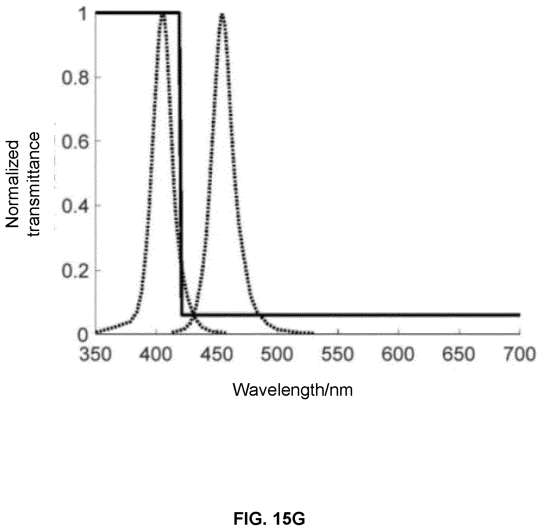

In some embodiments, the second optical surface 2021A on the third dichroic mirror may have a third dichroic film. FIG. 15G is a schematic diagram illustrating a transmittance spectrum of an exemplary third dichroic mirror according to some embodiments of the present disclosure. As shown in FIG. 15G, the third dichroic film may have a partial reflection characteristic of low reflection less than or equal to 10% and a transmission characteristic of high transmission greater than or equal to 90% to a violet light emitted by a UV_LED. The second optical surface 2022B on a second dichroic mirror may have a second dichroic film, which may have a partial reflection characteristic of low reflection less than or equal to 10% and a transmission characteristic of high transmission greater than or equal to 90% to a green light emitted by a G_LED. The second optical surface 201A on a first dichroic mirror may have a third dichroic film, which may have a partial reflection characteristic of low reflection less than or equal to 10% and a transmission characteristic of high transmission greater than or equal to 90% to a red light emitted by an R_LED.

During use, a light flux less than or equal to 10% of the violet light emitted by the light source 1021 (e.g., the UV_LED) of the at least one second light source 102 may be reflected by the second optical surface 2021A on the third dichroic mirror, and part of the reflected light may enter the corresponding first photodetector 81; a light flux of less than or equal to 10% of the green light emitted by the light source 1023 (e.g., the G_LED) of the at least one second light source 102 may be reflected by the second optical surface 2022B on the second dichroic mirror, and part of the reflected light may enter the corresponding third photodetector 83. A light flux of less than or equal to 10% of the red light emitted by the first light source 101 (e.g., the R_LED) may be reflected by the second optical surface 201A on the first dichroic mirror, and part of the reflected light may enter the corresponding fourth photodetector 84, thereby realizing the detection of the light flux of the UV_LED, the G_LED and the R_LED.

The second optical surface 2021A on the third dichroic mirror, the second optical surface 2022B on the second dichroic mirror, and the second optical surface 201A on the first dichroic mirror of the present application can reflect less than or equal to 10% of the light flux, or reflect less than or equal to 5% of the light flux. According to the photosensitivity characteristic of the photodetectors 81-84, the device can ensure that the detection light flux is maintained at an appropriate level without sacrificing too much effective illumination light.

In some embodiments, when the light source device 100 includes a plurality of dichroic mirrors, the same beam splitting film may be arranged on the second optical surface of each of the plurality of dichroic mirrors. The same beam splitting film may be configured to split the light reflected by the second optical surface. When a waveband of the light reflected by the second optical surface is different, a beam splitting wavelength of the same beam splitting film may cover the wavelengths of the lights of different wavelengths. For example, the second optical surface 201A on the first dichroic mirror, the second optical surface 2022B on the second dichroic mirror, and the second optical surface 2021A on the third dichroic mirror may be respectively provided with the same optical film. The optical film may be a broadband beam splitting film. Meanwhile, the optical film may at least cover a broadband (e.g., 370 nm-650 nm) of the light emitted by the UV_LED, the G_LED, and the R_LED, and may have a good consistency of the partial reflection characteristic of less than or equal to 10% of low reflection and the transmission characteristic of greater than or equal to 90% of high transmission in a broadband range of 370 nm-650nm. The same optical film is used in the embodiments of the present disclosure, which simplifies the process and reduces the system cost.

In some embodiments, the at least one light flux measurement module may further include an aperture diaphragm. For example, the aperture diaphragm may be provided at a front end of the first photodetector 81, the second photodetector 82, the third photodetector 83, and the fourth photodetector 84, respectively. A magnitude of the detection light incident into the first photodetector 81, the second photodetector 82, the third photodetector 83, and the fourth photodetector 84 may be adjusted by limiting a size of the aperture diaphragm, so as to achieve a balance between the detection sensitivity and the maximum detection saturation light, and realize light monitoring of a high dynamic range.

In some embodiments, as shown in FIG. 15F, the at least one light flux measurement module may further include a background light detector 8A. A position of the background light detector 8A may correspond to the position of the second photodetector 82. For example, the background light detector 8A may be provided to eliminate the influence of a background stray light on a measurement result of the second photodetector 82. The background light detector 8A may be provided on one side of the second photodetector 82, and the background light detector 8A can hardly receive the beam of the detection light transmitted by the B_LED through the first optical zone R1. The background light detector 8A may be located outside a range covered by the corresponding beam of the detection light on the corresponding light flux measurement module. By subtracting a detection signal of the second photodetector 82 from a background light signal detected by the background light detector 8A, a B light detection signal that is more consistent with an output B light may be obtained, thereby achieving the control of the B light flux with higher precision.

In some embodiments, in order not to increase the use of the background light detector 8A, the first photodetector 81 may be used as a background light detection photoelectric detector, which basically cannot receive the B light detection beam, thereby simplifying the system.

In some embodiments, as shown in FIG. 15F, the at least one light flux measurement module may be further provided with a background light detection photoelectric detector 8B. The background light detection photoelectric detector 8B may be located outside a diameter of a UV light detection beam obtained by reflecting an approximately collimated beam through the second optical surface 201A on the first dichroic mirror. That is, the background light detection photoelectric detector 8B hardly receives the UV light reflected by the second optical surface 201A on the first dichroic mirror. By subtracting the detection signal of the first photodetector 81 from the background light signal detected by the background light detection photoelectric detector 8B, a UV light detection signal that is more consistent with the output UV light may be obtained, thereby achieving the control of UV light flux with higher precision. Similarly, corresponding background light detection photoelectric detectors may be arranged for the third photodetector 83 and the fourth photodetector 84 to eliminate the influence of the background stray light and improve the detection accuracy, which is not repeated here.

In some embodiments, the at least one light flux measurement module may further include a filter. In order to achieve high-precision control of the hue stability and brightness of the illumination light, the spectrum of the detection beam of one or more the photodetectors may be spectrally filtered. For example, the filter may be provided on front ends of the first photodetector 81, the second photodetector 82, the third photodetector 83, and the fourth photodetector 84, respectively. In some embodiments, in order to perform spectral filtering on the spectrum of the detection beam of the first photodetector 81, the second photodetector 82, the third photodetector 83, and the fourth photodetector 84, the filter may be configured in a measurement optical path of the first photodetector 81, the second photodetector 82, the third photodetector 83, and the fourth photodetector 84, respectively, to cut off a portion exceeding a spectral range of the output illumination light to achieve a measurement spectrum that is consistent with or similar to the output spectra B1-B4 of the light source 1021 of the at least one second light source 102, the light source 1022 of the at least one second light source 102, the light source 1023 of the at least one second light source 102, and the first light source 101.

In some embodiments, the accuracy of light flux detection is ensured by the correspondence between the light flux detection by the first photodetector 81, the second photodetector 82, the third photodetector 83, and the fourth photodetector 84 and the strong correlation between the output components of the second light source 1021, the second light source 1022, the second light source 1023, and the first light source 101 in the output light, thereby maintaining the hue stability of the illumination light and the stability of the light flux, and simplifying the light flux control strategy.

In some embodiments, a bandpass filter L3 with a transmission characteristic within a spectrum B3 range may be provided at the front end of the third photodetector 83 to effectively filter out the blue excitation light in the spectrum of the fluorescent G_LED, maintaining the spectrum of the G_LED detection beam similar to or consistent with an output spectrum B3; or, a short-wave pass or bandpass filter L1 with a transmission characteristic within a spectrum B1 range may be provided at the front end of the first photodetector 81. A bandpass filter L2 with a transmission characteristic within a spectrum B2 range may be provided at the front end of the second photodetector 82. A long-wave pass or bandpass filter L4 with a transmission characteristic within a spectrum B4 range may be provided at the front end of the fourth photodetector 84.

In some embodiments, by designing the first optical surface and the second optical surface of the dichroic mirror with different optical properties, the light combination of a plurality of light sources is completed, and light splitting detection of the light flux of each light source is realized. The detection beam is obtained without adding additional optical elements (e.g., a beam-splitting reflector or other beam splitting optical elements) to realize light splitting detection. The detection beam may be a small amount of reflected and transmitted light through the first optical surface or the second optical surface of the dichroic mirror. The system design and the feedback control strategy can be simplified.

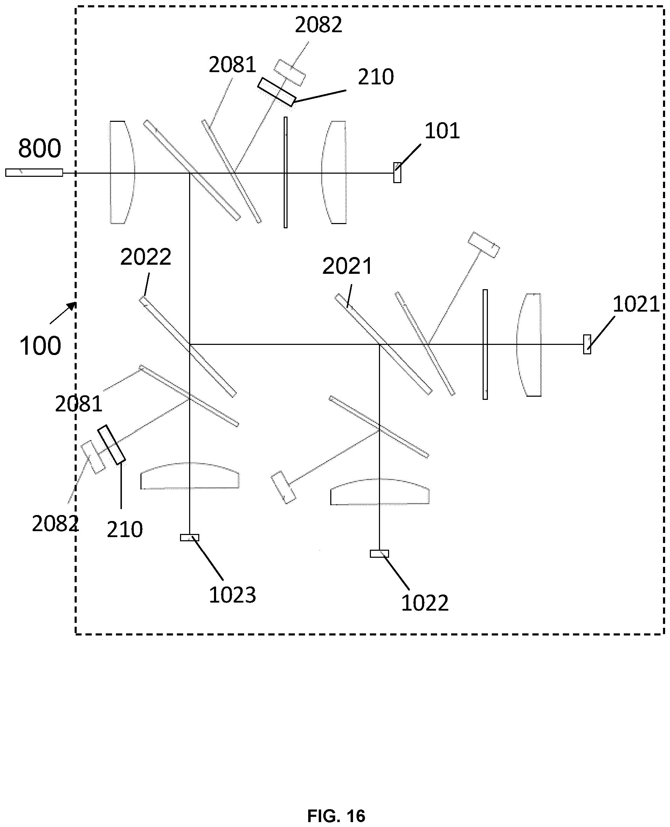

FIG. 16 is a schematic structural diagram illustrating an exemplary light source device according to some embodiments of the present disclosure. The difference between the light source device 100 shown in FIG. 16 and the light source device 100 shown in FIG. 15A is that the light combination module further includes a third filter 210 corresponding to the at least one light flux measurement module 208. In some embodiments, the third filter 210 may be disposed between the corresponding beam splitter 2081 and the photodetector 2082 to transmit a beam of a third target waveband and/or a fourth target waveband. For example, the at least one light flux measurement module 208 may be disposed in an optical path of each of the at least one second light source 102, and the third filter 210 may be disposed between each beam splitter 2081 and the corresponding photodetector 2082. For the light source 1022 (e.g., a green fluorescent light source), the third filter 210 may be a bandpass filter with a transmission characteristic within a spectral range of the light source 1022 (e.g., the green fluorescent light source), and may be configured to effectively filter out a blue laser emission in a spectrum of the light source 1022 (e.g., the green fluorescent light source), to ensure that a detection spectrum measured by the photodetector 2082 is similar to or consistent with an output spectrum of the light source 1022 (e.g., the green fluorescent light source) in a combined light. For the light source 1021 (e.g., a blue light source), the third filter 210 may be disposed between the photodetector 2082 and the beam splitter 2081. The third filter 210 may be a bandpass filter with a transmission characteristic within a spectral range of the light source 1021 (e.g., the blue light source), and may be configured to ensure that the detection spectrum measured by the photodetector 2082 is similar to or consistent with an output spectrum of the light source 1021 (e.g., blue light source) in the combined light. For the light source 1023 (e.g., a red light source), the third filter 210 may be disposed between the photodetector 2082 and the beam splitter 2081. The third filter 210 may be a long-wave pass or bandpass filter with a transmission characteristic within a spectral range of the light source 1023 (e.g., the red light source), and may be configured to ensure that the detection spectrum measured by the photodetector 2082 is similar to or consistent with an output spectrum of the light source 1023 (e.g., the red light source) in the combined light.

In some embodiments, in order to ensure that the detection spectrum obtained through the third filter 210 is consistent with or similar to the output spectrum of the first light source 101 in the combined light, a difference between the third target waveband and a first target waveband of a first beam in the combined light may be less than a first preset difference threshold. In some embodiments, in order to ensure that the detection spectrum obtained through the third filter 210 is consistent with or similar to the output spectrum of each of the at least one second light source 102 in the combined light, a difference between the fourth target waveband and a second target waveband of a second beam in the combined light may be less than a second preset difference threshold. In some embodiments, the first preset difference threshold and/or the second preset difference threshold may not be greater than 10 nm, respectively. For example, a wavelength of the first target waveband of the first beam in the combined light may be in a range of 390 nm-410 nm. That is, a short-wave portion of the first target waveband of the first beam may be 390 nm and a long-wave portion of the first target waveband of the first beam may be 410 nm, then a short-wave portion of the third target waveband may be within a range of 380 nm - 400nm, and a long-wave portion of the third target waveband may be within a range of 400 nm-420 nm.

In some embodiments, in order to achieve high-precision control of the color hue stability and the brightness of the illumination light, spectral filtering may be performed on a third beam and a fourth beam incident into the photodetector 2082. For example, the third filter 210 may be disposed in a measurement optical path of the photodetector 2082 (e.g., disposed between the beam splitter 2081 and the photodetector 2082) to cut off a non-effective output spectrum of the output illumination light.

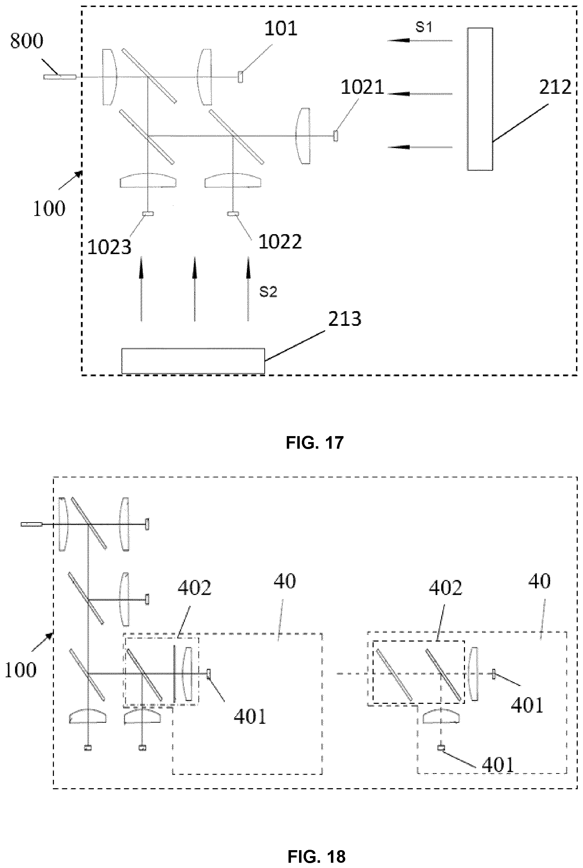

In some embodiments, the heat generated by the light source during operation causes a junction temperature (e.g., a PN junction temperature) to increase, and the relevant parameters (e.g., an amount of light and a spectrum of the light source) of each light source are easily affected by the operation temperature. The increase in the junction temperature causes a peak wavelength to drift, and as the junction temperature increases, the light flux decreases, which is particularly obvious for the R_LED. Therefore, it is necessary to control the heat dissipation of the light source device of the endoscope to maintain the light source device operating in a reasonable temperature range. FIG. 17 is a schematic structural diagram illustrating an exemplary light source device according to some embodiments of the present disclosure. As shown in FIG. 17, the light source device 100 may further include a first heat dissipation module 212 and a second heat dissipation module 213 for heat dissipation from the light source device 100. In some embodiments, a heat dissipation direction of the first heat dissipation module 212 may be parallel to an optical axis of an output light, and a heat dissipation direction of the second heat dissipation module 213 may be perpendicular to the optical axis of the output light.

In some embodiments, the first heat dissipation module 212 and/or the second heat dissipation module 213 may include one or more fans disposed in an interior or exterior space of the light source device 100 for air cooling. As shown in FIG. 17, the first light source 101, and the light source 1021, the light source 1022, and the light source 1023 of the at least one second light source 102 may be arranged in two mutually perpendicular or approximately perpendicular directions, respectively. A heat dissipation direction S1 of the first heat dissipation module 212 and/or the heat dissipation direction S2 of the second heat dissipation module 213 may be determined according to the arrangement characteristic of each light source of the light source device 100. For example, the heat dissipation direction of the first heat dissipation module 212 may be parallel to the optical axis of the output light of the light guide module 800; the heat dissipation direction of the second heat dissipation module 213 may be perpendicular to the optical axis of the output light of the light guide module 800. In some embodiments, a first heat dissipation fan and a second heat dissipation fan may be respectively arranged in the first heat dissipation direction S1 and the second heat dissipation direction S2 for overall heat dissipation of the light source device 100 and/or other components (e.g., the control module 500) of the endoscope system 10. A good comprehensive heat dissipation effect may be achieved by optimizing the air duct design and using a limited count of fans. In some embodiments, when heat dissipation is performed on the light source device 100, the first heat dissipation module 212 and/or the second heat dissipation module 213 may also be combined in various ways for heat dissipation. For example, heat dissipation may be performed on the light source device 100 by conduction using a heat conductive adhesive, a heat conductive sheet, heat dissipation fins, water cooling, liquid cooling, or the like.

FIG. 18 is a schematic structural diagram illustrating an exemplary light source device according to some embodiments of the present disclosure. As shown in FIG. 18, the light source device 100 may further include a light source extension interface. The light source extension interface may be configured to connect an extension module 40. In some embodiments, the extension module 40 may include at least one third light source 401 and at least one second light combination module 402 corresponding to the at least one third light source. In some embodiments, the at least one second light combination module 402 corresponding to the at least one third light source 401 may include a third light combination element. The third light combination element may be configured to reflect and/or transmit a fifth beam emitted by the at least one third light source 401 to realize light combination in sequence, thereby forming a second incident beam incident onto the at least one second light combination element for reflection or transmission. In some embodiments, the corresponding light source extension interface may be reserved on the light source device 100, and the extension module 40 may be connected through the light source extension interface to realize various lighting requirements of the endoscope system 10 at a low cost. More importantly, the interface may be reserved for subsequent new lighting requirements, maintaining the inheritance of the light source device 100 of the endoscope system 10. In some embodiments, the at least one third light source 401 may include a red light source. In some embodiments, the at least one third light source 401 may include the red light source and an amber light source. In some embodiments, the at least one third light source 401 may include the red light source, a first infrared light source, and a second infrared light source.

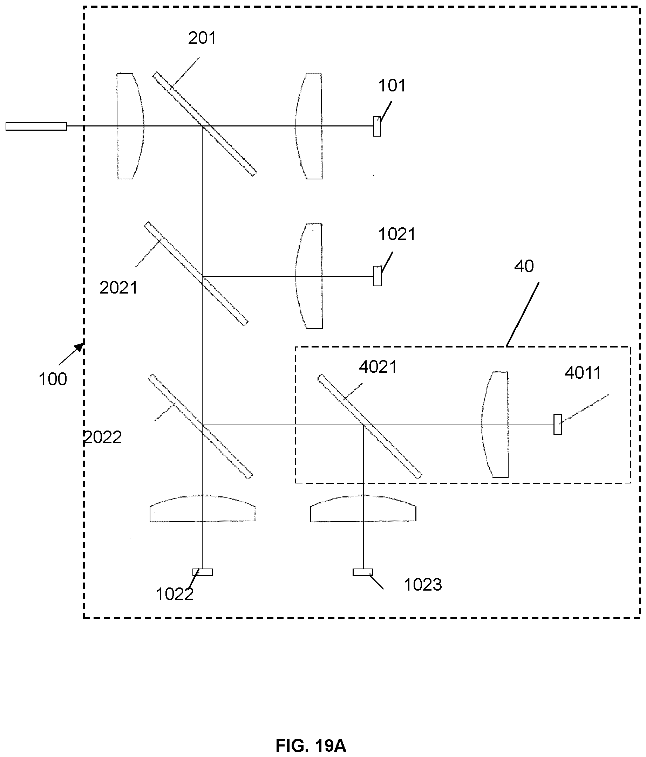

FIG. 19A is a schematic structural diagram illustrating an exemplary light source device according to some embodiments of the present disclosure. In some embodiments, as shown in FIG. 19A, the extension module 40 may include at least one light source 401 and at least one second light combination module 402 corresponding to the at least one light source. In some embodiments, in order to realize a special light of a bleeding point observation mode, the at least one light source 401 may include an amber light source 4011. At least one light combination module corresponding to the at least one light source may include a third light combination element 4021. In some embodiments, a fifth beam emitted by the amber light source 4011 may be converted into a parallel beam through a collimating lens. The third light combination element 4021 may transform the parallel beam into a second transmitted light, and reflect a second beam emitted by the second light source 1023 corresponding to the third light combination element 4021 to generate a second reflected light. The second reflected light, the second transmitted light, and a second beam other than the second beam emitted by the second light source 1023 may be reflected and/or transmitted by the at least one second light combination element 202 (e.g., the light combination element 2021 and the light combination element 2021) to generate a first incident beam incident onto the first light combination element 201, such that the amber light source 4011 is merged into the beams of the above four light sources for light combination and extended integration. In some embodiments, the collimating lens, a second filter, at least one light flux measurement module, etc., may also be provided between the amber light source 4011 and the third light combination element 4021.

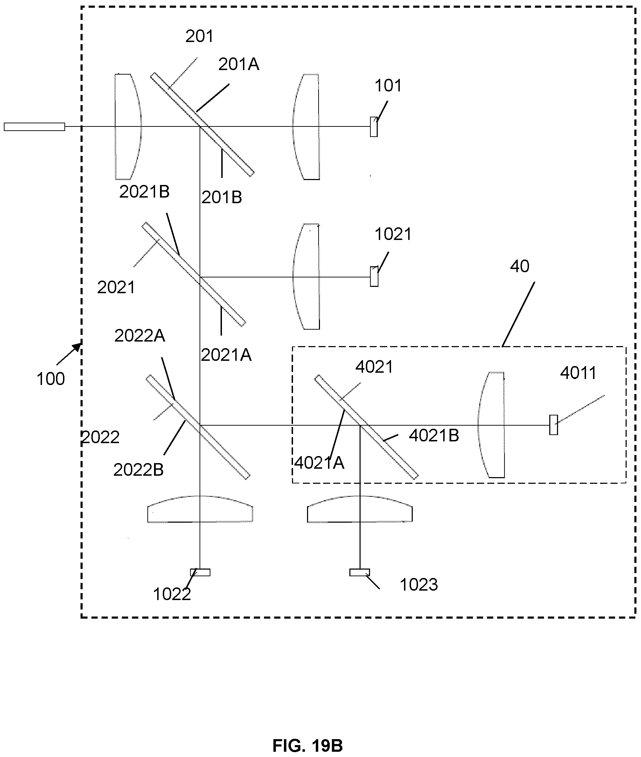

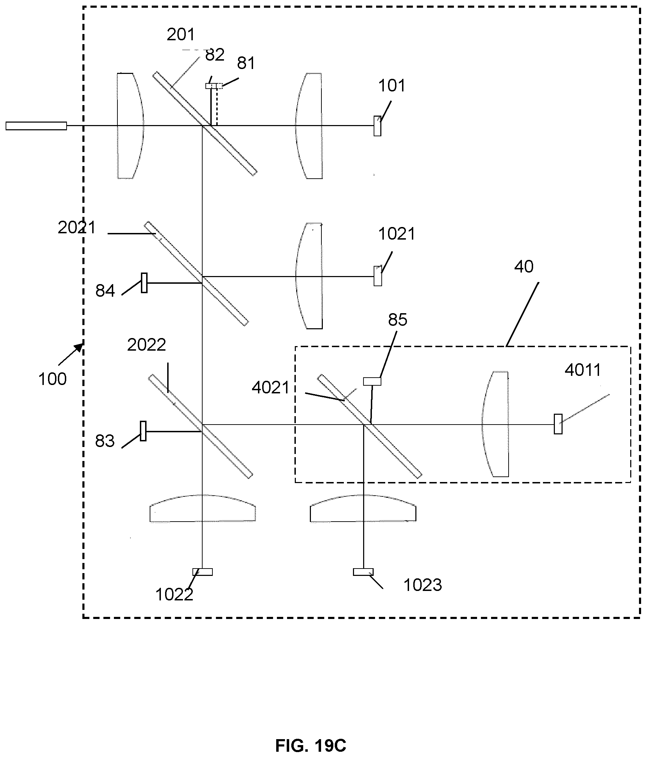

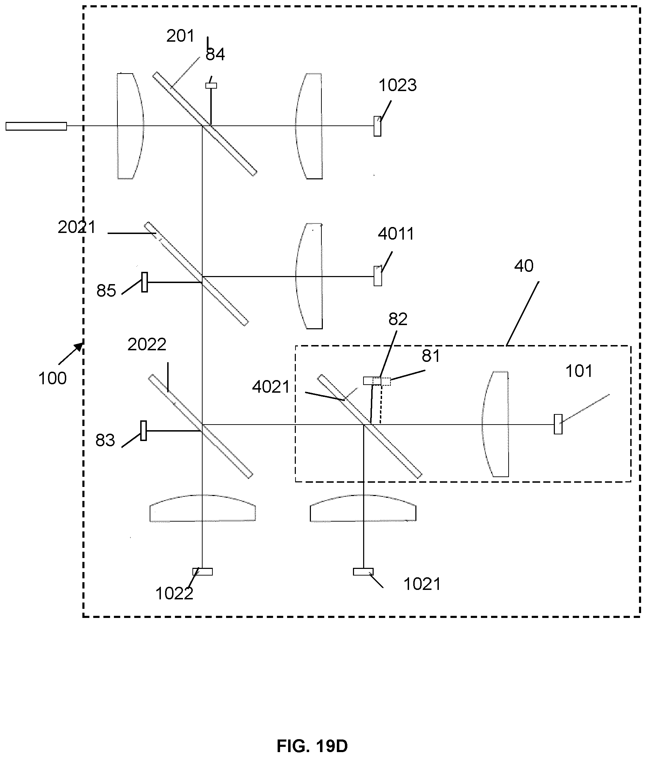

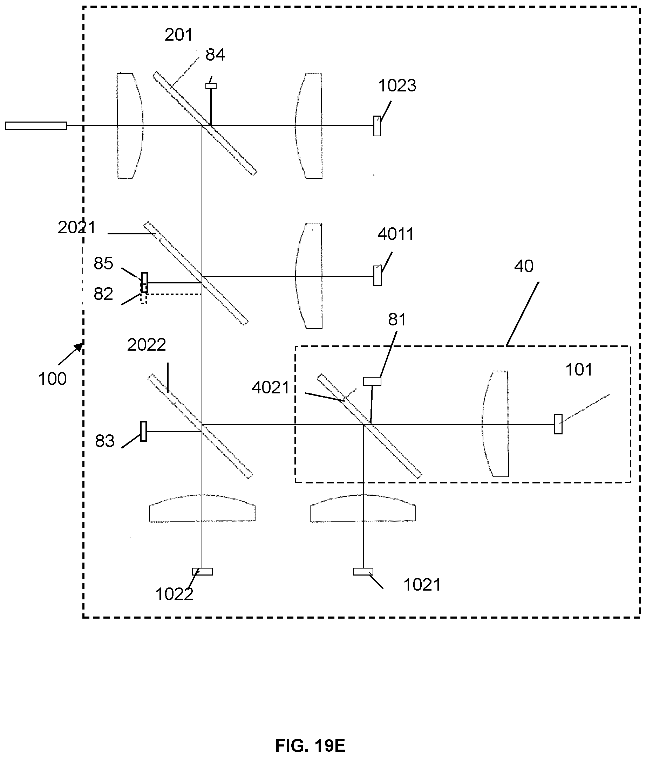

FIG. 19B is a schematic structural diagram illustrating an exemplary light source device according to some embodiments of the present disclosure. FIG. 19C is a schematic structural diagram illustrating an exemplary light source device according to some embodiments of the present disclosure. FIG. 19D is a schematic structural diagram illustrating an exemplary light source device according to some embodiments of the present disclosure. FIG. 19E is a schematic structural diagram illustrating an exemplary light source device according to some embodiments of the present disclosure. Similar to the light combination element described in FIG. 15B and FIG. 15C as a dichroic mirror, the light combination element of the light source device 100 shown in FIG. 19A may also be a dichroic mirror. On the basis of the light source device 100 described in FIG. 19A, in the light source device 100 shown in FIGs. 19B-19E, the first light combination element 201, the light combination element 2021 and the light combination element 2021 of the at least one second light combination element 202, and the third light combination element 4021 may be a first dichroic mirror, a second dichroic mirror, a third dichroic mirror, and a fourth dichroic mirror, respectively. In addition, the light source device 100 shown in FIGs. 19C-19E may further include the first photodetector 81, the second photodetector 82, the third photodetector 83, the fourth photodetector 84, and the fifth photodetector 85 for measuring a light flux entering therein.

In some embodiments, the first light source 101, the light source 1021, the light source 1022, and the light source 1023 of the at least one second light source 102, and the light source 4011 may be an ultraviolet (UV-LED) light source, a blue (B-LED) light source, a green (G-LED) light source, a red (R-LED) light source, and an amber (A-LED) light source, respectively. On the basis of FIGs. 15A-15C, the amber light source 4011 may be added to the light source device 100 shown in FIGs. 19A-19E. A peak wavelength of the amber light source 4011 may be in a range of 590 nm-610 nm. The absorption degree of hemoglobin to light has a large variation around 600 nm. A peak wavelength of the first light source 101 (e.g., the R_LED) may be in a range of 620 nm- 640 nm. Compared with the light of 600 nm wavelength, an absorption coefficient may be small, and a scattering coefficient of a tissue may also be small, which is conducive to improving the visibility of deep blood vessels. Other structures may be the same as the above, which are not repeated here.

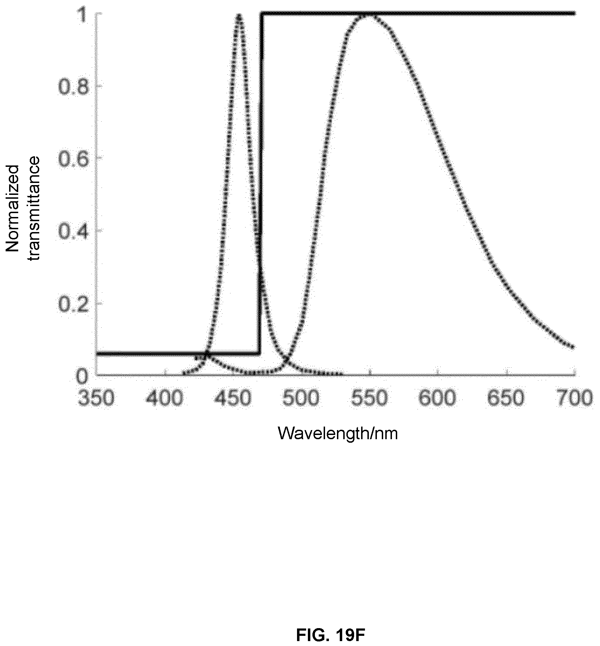

In some embodiments, referring to FIG. 19B and FIG. 19C, a transition zone wavelength of the first optical surface 201B on the first dichroic mirror may be in a range of 410 nm-430 nm, and a transmittance to an ultraviolet light (≤ 420 nm) emitted by the UV_LED may reach an optimal transmittance T1 according to a coating process. Preferably, T1 ≥ 97%. Meanwhile, for a blue light (≥ 420 nm) emitted by the B_LED, the transition zone wavelength of the first optical surface 201B on the first dichroic mirror may have a light splitting characteristic with a reflection characteristic as the dominant portion and a transmission characteristic as a supplemented portion. In some embodiments, a beam splitting film may be provided on the first optical surface of the dichroic mirror. In some embodiments, the beam splitting film may be configured to transmit the light emitted by the corresponding light source while performing optical path integration during reflection, such that the transmitted light enters the corresponding light flux measurement module. FIG. 19F is a schematic diagram illustrating a spectrum curve of a first optical surface of a dichroic mirror according to some embodiments of the present disclosure. As shown in FIG. 19F, a reflectivity RR and a transmittance T2 of the first optical surface 201B in a waveband above 430 nm may be designed with a transmittance characteristic of less than or equal to 10 % for B light, and a reflection characteristic of greater than or equal to 90 % for B light through a beam splitting film. A coating characteristic may be highly transmitting to a violet light emitted by the UV_LED, and reflect greater than or equal to 90% of a blue light emitted by the B_LED while transmitting less than or equal to 10%, such that the light emitted by the UV_LED and the light emitted by the B_LED are combined, and transmittance splitting of the B_LED is also realized to enter the second photodetector 82 as the detection light to detect the output light flux of the B_LED. Optionally, a beam splitting film may be provided on the first optical surface of the dichroic mirror. The beam splitting film may be configured to reflect the light emitted by the corresponding light source while performing optical path integration during transmission, such that the reflected light enters the corresponding light flux measurement module. The light emitted by the R_LED may be reflected by the first optical surface 4021A of the fourth dichroic mirror, reflected by the first optical surface 2022B of the third dichroic mirror, and then reflected by the second optical surface 2021A of the second dichroic mirror in sequence to obtain an R detection beam, which enters the fourth photodetector 84 as the detection light to detect the output light flux of the R_LED. The optical path integration of the G_LED may be performed before the optical path integration of the B_LED. The first optical surface 2021A of the second dichroic mirror used as the optical path integration of G_LED and B_LED realizes the cutoff of the blue excitation light in the light emitted by the G_LED, and has the characteristics of completely cutting off the blue excitation light in the reflected light of the G_LED and completely reflecting the light emitted by the B_LED. That is, the reflectivity of the blue light emitted by the B_LED reaches the highest according to the coating process, and almost has no light splitting characteristics of transmitting the blue light.

The difference between FIG. 19D and FIG. 19E and FIGs. 19A-FIG. 19C lies in the positions of the light sources. The first light source 101, the light source 1021, the light source 1022, and the light source 1023 of the at least one second light source 102, and the light source 4011 may be an ultraviolet (UV-LED) light source, a blue (B-LED) light source, a green (G-LED) light source, a red (R-LED) light source, and an amber (A-LED) light source, respectively. The optical path integration of the G_LED may be performed after the optical path integration of the B_LED. Further, the optical path integration may be performed on the light sources according to an ascending order of wavelengths. Therefore, the first optical surface 201B on the first dichroic mirror, the first optical surface 2022A on the second dichroic mirror, the first optical surface 2021B on the third dichroic mirror, and the first optical surface 4021A on the fourth dichroic mirror may have a long-wave pass or short-wave pass characteristic, which has the characteristics of simplifying the coating process and reducing the system cost.

In some embodiments, the characteristics of the first optical surface 2021B on the third dichroic mirror and the first optical surface 4021A on the fourth dichroic mirror are shown in FIG. 10 and FIG. 6. The characteristics of the first optical surface 2022A on the second dichroic mirror are shown in FIG. 10. The first optical surface 201B on the first dichroic mirror may have a long-wave pass characteristic with a transition zone wavelength of about 600 nm-630 nm, which transmit the light of the R_LED above 610 nm and reflects the light of the A-LED below 610 nm, so as to complete the optical path integration of the violet light, the blue light, the green light, and the amber light emitted by the UV_LED, the B_LED, the G_LED, and the A_LED, and the red light emitted by the R_LED.

In some embodiments, with the beam splitting and spectral characteristics of the second optical surfaces 201A, 2021A, and 4021 B of the first dichroic mirror, the third dichroic mirror, and the fourth dichroic mirror, the light emitted by the R_LED, the G_LED, and the UV_LED may be partially split to enter the light flux measurement module (e.g., the fourth photodetector 84, the third photodetector 83, and the first photodetector 81) corresponding to each LED. With the spectral characteristics of the second optical zone R2 of the first optical surface 2022A on the second dichroic mirror, the light emitted by the A_LED may be transmitted and split through the second optical zone R2 to enter the fifth photodetector 85. With the spectral characteristics of the second optical zone R2 of the first optical surface 4021A on the fourth dichroic mirror, or the spectral characteristics of the first optical surface 4021A on the fourth dichroic mirror, the light emitted by the B_LED may be transmitted and split to enter the second photodetector 82, thereby achieving spectral detection of each LED while realizing the optical path integration.

In some embodiments, the first optical surfaces 2021B and 402A respectively corresponding to the second dichroic mirror and the fourth dichroic mirror may not be coated, or may have the same coating characteristic (e.g., the first optical surfaces 2021B and 402A have a beam splitting film that transmits about 95% of the amber light emitted by the A_LED and about 5% of the blue light emitted by the B_LED, or have an antireflection film with an antireflection property). In some embodiments, the second optical areas R2 of the first optical surfaces 2021B and 402A respectively corresponding to the second dichroic mirror and the fourth dichroic mirror may be coated in the same batch, thereby simplifying the coating process and reducing the system cost.

In some embodiments, the second photodetector 82 may be offset, and the first photodetector 81 and the second photodetector 82 may be arranged vertically side by side or in a left-right direction in a detection optical path space shown in FIG. 19D to optimally receive the detection light of the light source 4011 and the light source 1023.

In some embodiments, as shown in FIG. 19E, for a spectral detection solution for the B light emitted by the B_LED, the second optical surface 2022B on the second dichroic mirror may be configured to have a beam splitting and spectral characteristic. The light emitted by the B_LED may be reflected by the first optical surfaces 4021A and 2021B on the fourth dichroic mirror and the third dichroic mirror in sequence to enter the second optical surface 2021A on the second dichroic mirror for spectral detection and observation of the B light, and enter the second photodetector 82 as the detection light to achieve the reflected spectral detection of the B light. In some embodiments, the second photodetector 822 may be offset, and the second photodetector 82 and the fifth photodetector 85 may be arranged vertically side by side or in the left-right direction in a detection optical path space shown in FIG. 19E.

In some embodiments, a ratio of the detection light received from the first photodetector 81 to the fifth photodetector 85 to the light emitted by each light source may be moderate. In this way, sufficient light may be achieved to meet the monitoring accuracy of the system, and the light is not excessive, which avoids the saturation of the photodetectors due to excessive detection light, and achieves a maximum dynamic detection range while performing high-accuracy and high-dynamic range detection on the light flux of each light source without too much loss of the effective output illumination light.

Meanwhile, the fluorescent G_LED may emit the green light and also have the blue excitation light. In order to prevent the blue excitation light of G_LED from being transmitted into the effective illumination optical path through a partitioned coating spectroscopy or a dichroic beam splitting characteristic of the dichroic mirror in a downstream optical path, the detection optical path may meet the following condition: the G_LED serves as the first optical surface of the dichroic mirror that integrates the optical path of G_LED and the optical path of B_LED to realize the cutoff of the blue excitation light in the light emitted by the G_LED, and has the characteristic of completely cutting off the blue excitation light in the light reflected by the G_LED and completely reflecting the light emitted by the B_LED. That is, the reflectivity of the blue light emitted by the B_LED reaches the highest according to the coating process, and almost has no spectral characteristic of transmitting the blue light.

The above condition restriction prevents the blue excitation light of the G_LED and the blue light emitted by the B_LED in the output illumination light from being confused with each other, such that the spectrum curves of the light sources in the output illumination light are independent of each other and have as little or almost no overlapping band. For the light source device 100 that uses other fluorescent LEDs for light combination, the detection optical path may have similar characteristics.

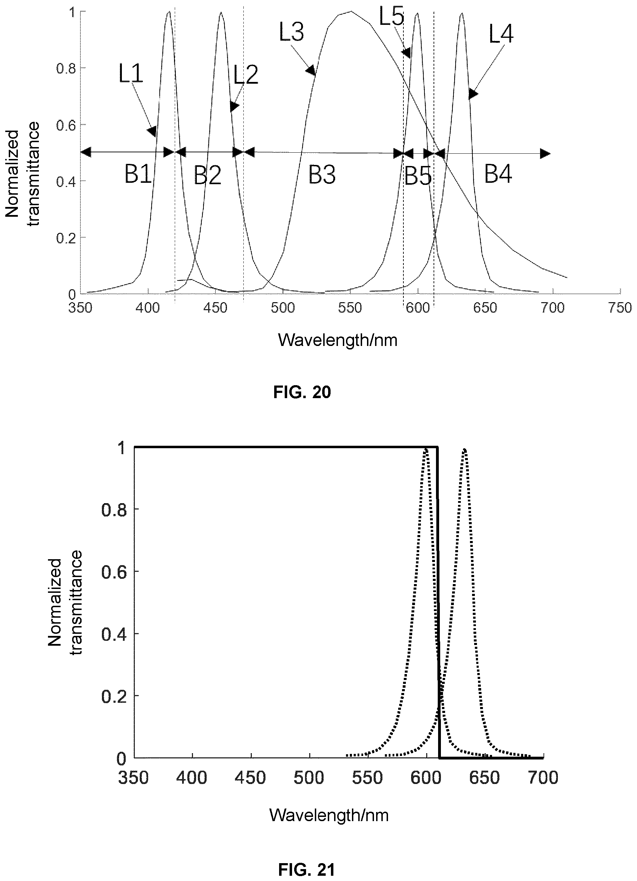

FIG. 20 is a schematic diagram illustrating exemplary spectrum curves of light sources according to some embodiments of the present disclosure. L1, L2, L3, L4 and L5 represent spectrum curves of the first light source 101, the light source 1021, the light source 1022, the light source 1023 and the amber light source 4011, respectively. Specifically, the first light source 101 may be a violet light source, the light source 1021, the light source 1022, and the light source 1023 may be a blue light source, a green light source, and a red light source, respectively, and the amber light source 4011 may emit an amber light. Referring to FIG. 19A and FIG. 20, when the first light source 101 is the violet light source that emits a short-wave spectrum, peak wavelengths of the light source 1021, the light source 1022, the light source 1023 and the amber light source 4011 may be longer than a peak wavelength of the violet light source. A second beam emitted by a second light source with a longer peak wavelength (e.g., the light source 1023 which is a red light source) may be reflected by the second light combination element 2022, and a second beam emitted by a second light source with a shorter peak wavelength (e.g.,, the light source 1022 which is a green light source) may be transmitted by the second light combination element 2022, thereby completing the light combination of the at least one second light source in sequence. The second light combination element 2022 may be a short-wave pass dichroic mirror.

FIG. 21 is a schematic diagram illustrating a transmittance spectrum of an exemplary third light combination element according to some embodiments of the present disclosure. As shown in FIG. 21, the third light combination element 4021 may have a short-wave pass characteristic with a transition zone wavelength of about 600 nm-620 nm, which transmits a spectral component of the amber light source 4011 below 610 nm and reflects a spectral component of the red light source above 610 nm. In some embodiments, the at least one second light combination element 202 (e.g., the light combination element 2021 and the light combination element 2022) may have different transition zone long-wave pass or short-wave pass characteristics. The third light combination element 4021 may transform a fifth beam emitted by the amber light source 4011 into a second transmitted light, and reflect a second beam emitted by the second light source 1023 corresponding to the third light combination element 4021 to generate a second reflected light. The at least one second light combination element 202 (e.g., the light combination element 2021 and the light combination element 2022) may reflect and/or transform the second transmitted light, the second reflected light, and the second beam emitted by the second light source 1023 corresponding to the third light combination element 4021 into a first incident beam that is incident onto the first light combination element 201. Finally, the first light combination element 201 may reflect the first incident beam and transmit the first beam emitted by the first light source to generate a combined light. Collimated light beams of each light source may be reflected and/or transmitted through the first light combination element 201, each of the at least one second light combination element 202, and the third light combination element 4021, such that the spectrum of each light source may be independent of each other, and independent spectrums may be obtained. That is, there is almost no portion where the wavelengths overlap with each other, which simplifies the control strategy for the proportion of each light source in the output light, and realizes high-precision control of the illumination light color hue and light flux stability.

In some embodiments, a peak wavelength of the amber light source 4011 may be in a range of 590 nm-610 nm. A spectral absorption coefficient of hemoglobin has a large variation around 600 nm. A peak wavelength of the light source 1023 (e.g., the red light source) may be in a range of 620 nm-640 nm. Compared with the spectrum of the amber light source 4011 of about 590 nm-610 nm, the spectrum of the light source 1023 (e.g., red light source) has a smaller absorption coefficient of hemoglobin and scattering coefficient of a living tissue. According to the difference in the absorption and scattering characteristics of the spectrum of the amber light source 4011 and the light source 1023 (e.g., the red light source) in the output light, the use of the amber light source 4011 and the light source 1023 (e.g., the red light source) for illumination is conducive to improving the visibility of deep blood vessels.

In some embodiments, a photodetector may be disposed in an optical path of the amber light source 4011 to detect a light flux emitted by the amber light source 4011. Optionally or additionally, a filter may be disposed between the photodetector and a beam splitter.

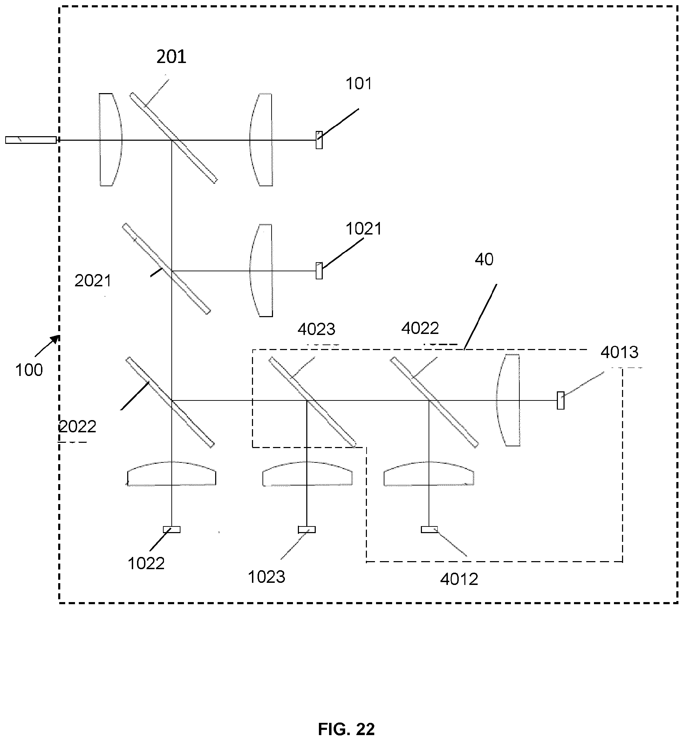

FIG. 22 is a schematic structural diagram illustrating an exemplary light source device according to some embodiments of the present disclosure. In some embodiments, the extension module 40 may include at least one light source and at least one light combination module corresponding to the at least one light source. In order to realize a special light of indocyanin green (ICG) fluorescence observation, as shown in FIG. 22, the at least one light source may include a first infrared light source 4012 and a second infrared light source 4013. The at least one light combination module corresponding to the at least one light source may include a fourth light combination element 4022 and a fifth light combination element 4023. In some embodiments, the fourth light combination element 4022 may be configured to reflect a sixth beam emitted by the first infrared light source 4012 to generate a second incident beam incident onto the fifth light combination element 4023, and transform a seventh beam emitted by the second infrared light source 4013 into a third transmitted light. In some embodiments, the fifth light combination element 4023 may be configured to transmit the second incident beam and the third transmitted light, and reflect a second beam emitted by the second light source 1023 corresponding to the fifth light combination element 4023 to generate a third incident beam incident onto the at least one second light combination element 202. In some embodiments, the at least one second light combination element 202 (e.g., the light combination element 2021 and the light combination element 2022) may be configured to reflect and/or transmit the third incident beam and a second remaining beam to form the first incident beam incident onto the first light combination element 201. The second remaining beam may include a second beam other than the second beam emitted by the second light source 1023 corresponding to the fifth light combination element 4023.

In some embodiments, the extension module 40 may be provided with a collimating lens between the first infrared light source 4012 and the fourth light combination element 4022 and a collimating lens between the second infrared light source 4013 and the fourth light combination element 4022. For example, a short-wave pass or band-pass filter may be added to an optical path of the first infrared light source 4012 and/or the second infrared light source 4013 to further highlight a narrowband characteristic of 800 nm-820 nm and 920 nm-940 nm. For example, a filter with a short-wave pass characteristic of a wavelength below 820 nm may be added to an optical path where the fourth light combination element 4022 and the first infrared light source 4012 are located, and a filter with a band-pass characteristic of 920 nm-940 nm may be added to an optical path where the fourth light combination element 4022 and the second infrared light source 4013 are located.

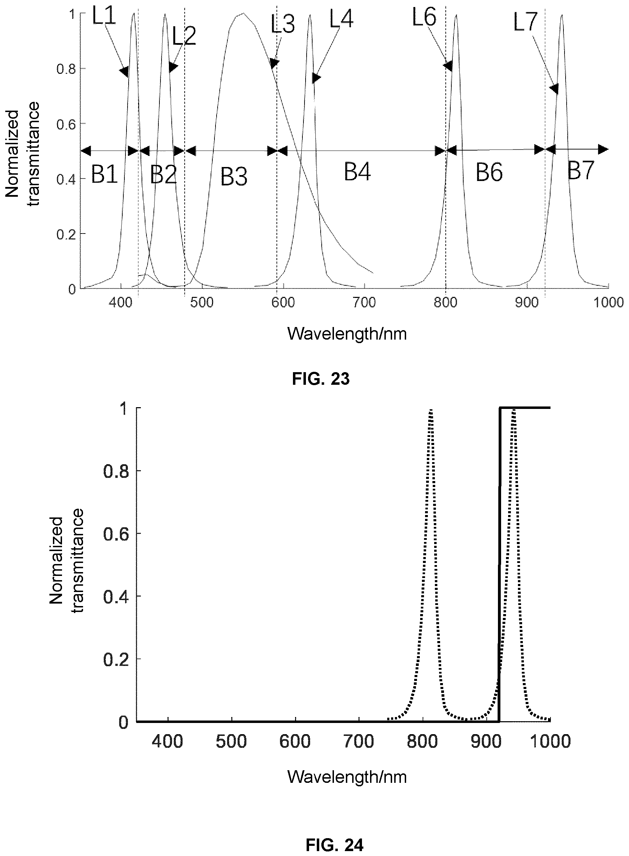

FIG. 23 is a schematic diagram illustrating exemplary spectrum curves of light sources according to some embodiments of the present disclosure. L1, L2, L3, L4, L6 and L7 represent spectrum curves of the first light source 101, the light source 1021, the light source 1022, the light source 1023, the first infrared light source 4012, and the second infrared light source 4013, respectively. Specifically, the first light source 101 can be a violet light source, and the light source 1021, the light source 1022 and the light source 1023 may be a blue light source, a green light source, and a red light source, respectively. In some embodiments, a wavelength of the first infrared light source 4012 may be in a range of 800 nm-830 nm. The second infrared light source 4013 may have a longer wavelength than the first infrared light source 4012. For example, a wavelength of the second infrared light source 4013 may be in a range of 910 nm-950 nm.

FIG. 24 is a schematic diagram illustrating a transmittance spectrum of an exemplary fourth light combination element according to some embodiments of the present disclosure. As shown in FIG. 24, the fourth light combination element 4022 may have a long-wave pass characteristic with a transition zone wavelength of about 910 nm-930 nm. The fourth light combination element 4022 may transmit a spectrum of a beam emitted by the second infrared light source 4013 that is higher than 920 nm, and reflects a spectrum of a beam emitted by the first infrared light source 4012 that is lower than 920 nm, so as to generate a first transmitted light.

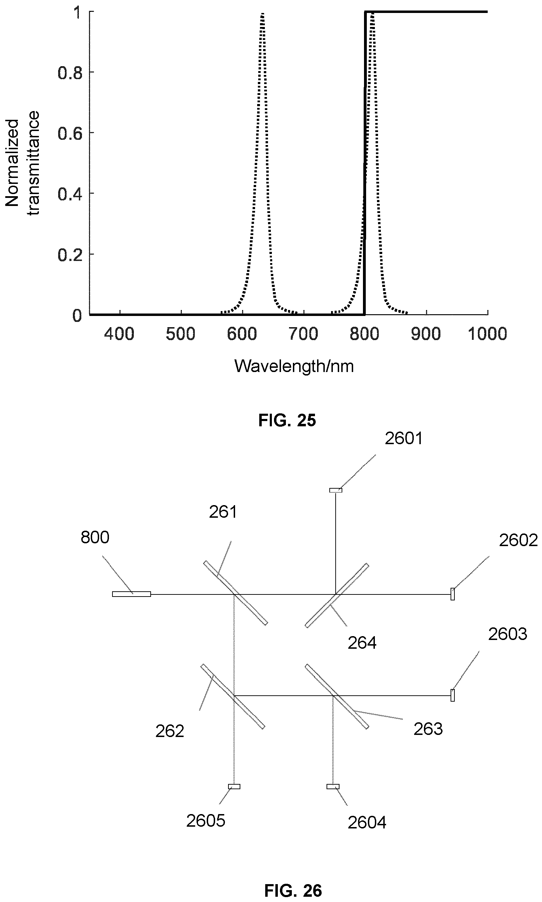

FIG. 25 is a schematic diagram illustrating a transmittance spectrum of an exemplary fifth light combination element according to some embodiments of the present disclosure. As shown in FIG. 25, the fifth light combination element 4023 may have a long-wave pass characteristic with a transition zone wavelength of about 790 nm-810 nm. The fifth light combination element 4023 may transmit a spectrum of the first transmitted light that is greater than 800 nm, and reflect a spectrum of the beam emitted by the light source 1023 (e.g., the red light source) that is less than 800 nm to generate a third incident beam.

In some embodiments, the light combination element 2021 and the light combination element 2022 of the at least one second light combination element 202 may have different transition zone long-wave pass or short-wave pass characteristics. The second light combination element 202 may reflect and/or transmit the third incident beam and a second beam other than the second beam emitted by the second light source corresponding to the fifth light combination element according to the transition zone long-wave pass or short-wave pass characteristics to generate a first incident beam incident onto the first light combination element 201. Finally, the first light combination element 201 may reflect the first incident beam and transmit a first beam related to the first light source to generate a combined light. The collimated light beams of each light source may be reflected and/or transmitted by the first light combination element 201, each of the at least one second light combination element 202, the fourth light combination element 4022, and the fifth light combination element 4023, so as to realize the mutual independence of the spectrum of each light source and obtain independent spectrums.

In some embodiments, a circuit interface may be provided on a circuit connected to the light source device 100. A circuit connection may be performed through the circuit interface to realize control of the extension module 40. It should be noted that the extension module 40 may meet various lighting requirements of the endoscope system 10 at a low cost through the extension or replacement process. More importantly, an interface may be reserved for subsequent new lighting requirements.

The light source device 100 shown in some embodiments of the present application may have an ordinary white light mode, a special light mode, and a hybrid light mode of white light illumination, which can realize the observation of the overall contour of the observed object, the emphasized observation of the blood vessels in the surface and middle layers, and the hybrid light observation image that considers the overall contour and the emphasized observation of the blood vessels, respectively. In some embodiments, the light source device 100 shown in some embodiments of the present disclosure may have an infrared light observation mode (e.g., a first infrared light source and a second infrared light source). After intravenous injection of ICG that easily absorbs infrared light, a clear observation image of the deep blood vessels and blood flow information of the mucosa is realized, or an interface is reserved for new special light/hybrid light illumination.

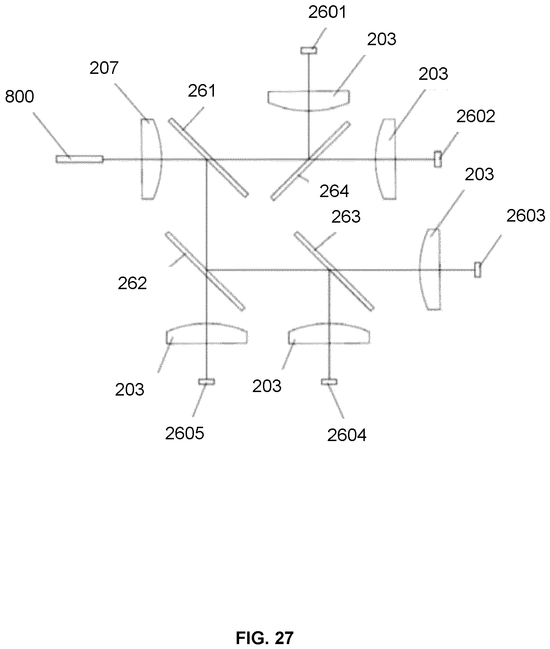

FIG. 26 is a schematic structural diagram illustrating an exemplary light source device according to some embodiments of the present disclosure. As shown in FIG. 26, the light source device 100 may include a first light source (e.g., a light source 2602), at least one second light source (e.g., a light source 2601, a light source 2603, a light source 2604, and a light source 2605), a first light combination element (e.g., light combination element 262), and at least one second light combination element (e.g., a light combination element 261, light combination element 263, and light combination element 264). The light guide module 800 may be connected with the light source device 100 and configured to output a combined light generated by the light source device 100. FIG. 27 is a schematic structural diagram illustrating an exemplary light source device 100 according to some embodiments of the present disclosure. The light source device 100 shown in FIG. 27 further includes one or more collimating lenses 203 on the basis of the light source device 100 shown in FIG. 26. In some embodiments, one of the one or more collimating lenses 203 may be disposed between one of the light sources and one of the light combination elements to convert a beam emitted by the light source into a parallel beam or an approximately parallel beam incident onto the corresponding light combination element.

In some embodiments, the light source 2601 may be a red light R_LED, the light source 2602 may be an amber light A_LED, the light source 2603 may be an ultraviolet light UV_LED, the light source 2604 may be a blue light B_LED, and the light source 2605 may be a green light G_LED. As shown in FIGs. 26-27, an angle between any two of the light combination element 261, the light combination element 262, and the light combination element 263 may be less than a seventh preset angle. In some embodiments, the seventh preset angle may be 5°, 10°, 15°, 20°, etc. For example, any two of the light combination element 261, the light combination element 262, and the light combination element 263 may be parallel. An angle between the light combination element 264 and any other light combination element (e.g., the light combination element 261, the light combination element 262, or the light combination element 263 ) may be greater than an eighth preset angle and less than a ninth preset angle. In some embodiments, the eighth preset angle may be 80°, 85°, etc., and the ninth preset angle may be 95°, 100°, etc. For example, the angle between the light combination element 264 and any other light combination element (e.g., the light combination element 261, the light combination element 262, or the light combination element 263) may be 90°.

In some embodiments, a spectrum curve of the amber light A_LED of the light source 2602 may be shown in FIG. 20. As shown in FIG. 20, the amber light A_LED may have a narrowband spectrum, and a peak wavelength of the amber light A_LED may be in a range of 590 nm-610 nm, and a bandwidth of the amber light A_LED may be about 20 nm. In some embodiments, the amber light A_LED may be a fluorescent conversion A_LED having a blue excitation light with a peak wavelength in a range of 430 nm-460 nm. The blue excitation light may excite a fluorescent material to generate a broadband light with a peak wavelength of about 590 nm-610 nm. A small amount of blue excitation light may not be absorbed by the fluorescent material and may be directly transmitted. That is, the broadband amber light emitted by a fluorescent_LED spectrum may include a small amount of blue excitation light in addition to the broadband spectrum with a peak wavelength of 590 nm-610 nm.

In some embodiments, the light combination element 264 may have a short-wave pass characteristic with a transition zone wavelength of about 600 nm-620 nm, which transmits the amber light and reflects the red light to realize combination of the red light and the amber light. The light combination element 263 may have a short-wave pass characteristic with a transition zone wavelength of about 400 nm-420 nm, which transmits a UV light and reflects a blue light, and realizes the combination of the UV light and the blue light. The light combination element 262 may have a long-wave pass characteristic with a transition zone wavelength of about 450 nm-470 nm, which transmits a green light and reflects the blue light and the UV light, and realizes the combination of the green light, the UV light, and the blue light. The light combination element 261 may have a short-wave pass characteristic with a transition zone wavelength of about 585 nm-605 nm, which transmits the amber light and the red light and reflects the green light, the blue light, and the UV light, and realizes the combination of the amber light, the red light, the green light, the blue light, and the UV light.

When the amber light A_LED is the fluorescent conversion A_LED, the light combination element 264 may perform long-wave cutoff filtering to cut off a spectrum of the broadband amber light that is above 610 nm. The light combination element 261 may perform a short-wave cutoff filtering to cut off a spectrum of the broadband amber light that is below 595 nm, thereby obtaining a narrowband amber light with a wavelength in a range of 590 nm-610nm or in a range of 595 nm-610 nm; or the light combination element 261 may cut off a spectrum of the broadband amber light that is below 590 nm to obtain a narrowband amber light with a peak wavelength in a range of 590 nm -610 nm.

When the amber light A_LED is the fluorescent conversion A_LED, long-wave cutoff or/and short-wave cutoff filtering may be performed using the light combination element, and a filter may be added at an appropriate position in an optical path of the broadband amber light A_LED to achieve a required filtering function. For example, the filter may be added between one of the one or more collimating lenses 203 and the light combination element 264 (as shown in FIG. 27). In some embodiments, the light source device may further include a filter switch-in and switch-out module. The filter switch-in and switch-out module may have two operation states in which the filter is switched in the optical path or switched out of the optical path, and may be configured to realize switching between a filter switch-in mode and a filter switch-out mode.

In some embodiments, the filter of the filter switch-in and switch-out module may be a filter for narrowband filtering a broadband G light emitted by the light source 2605. The filter may be disposed between the light combination element 261 and the light combination element 262 in the optical path of the light source 2605. The light source 2605 (e.g., the green light source) may emit a green light by exciting a fluorescent material with a blue LED (e.g., a fluorescent G_LED), with a peak wavelength of 510 nm-560 nm. A bandwidth of the light source 2605 may be in a range of 90 nm-110 nm. The filter may filter the broadband green light to obtain a narrowband green light of 520 nm-550 nm or 530 nm-550 nm, so that the light source 2605 has two output states of the broadband green light and the narrowband green light. In some embodiments, when the filter is switched out of the optical path through the filter switch-in and switch-out module, the light source device may have the white light mode; when the filter is switched in the optical path, the light source device has a bleeding point observation mode. The white light observation mode may be achieved by setting the light sources 2601-2605 to output in a certain proportion. The light source 2605 may output the broad band green light to obtain the white light mode, so as to observe the overall properties of the surface of a living tissue. The bleeding point observation mode may be achieved by setting the light source 2605 (e.g., the G-LED), the light source 2602 (e.g., the A-LED), and the light source 2601 (R-LED) that output a narrowband spectrum as a main output. The output spectrum of the light source 2602 (e.g., the A-LED) is easily absorbed by hemoglobin in the blood than the output spectrum of the light source 2601 (e.g., the R-LED). The difference in the absorption characteristics of the output spectrum of the light source 2602 and the output spectrum of the light source 2601 improves the visibility of deep blood vessels, and is mainly used for emphasizing the display of deep blood vessels or obtaining a bleeding point display image.

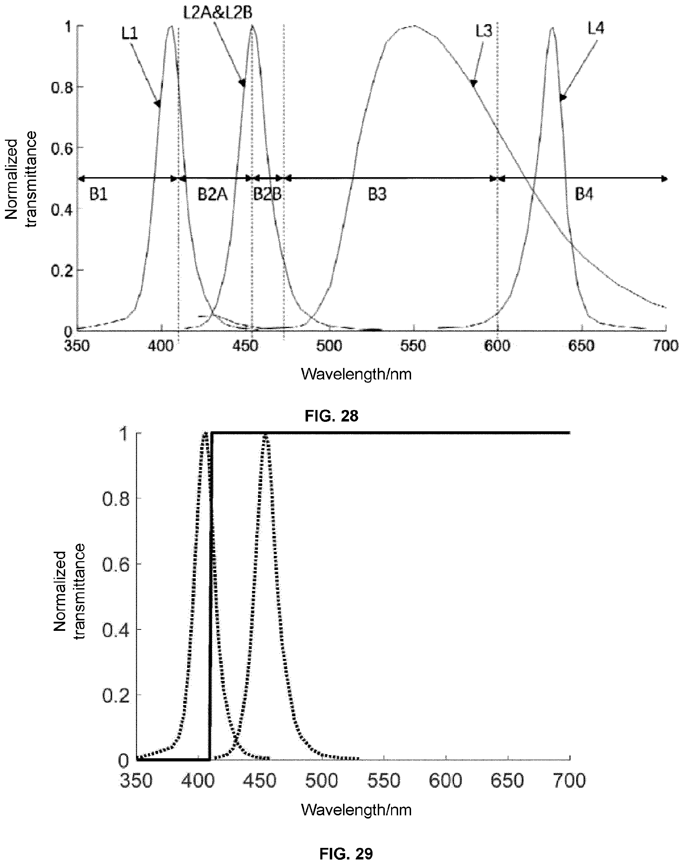

In some embodiments, the light source 2601 may be an ultraviolet light UV_LED, the light source 2602 may be a first blue light B_LED, the light source 2603 may be a second blue light B_LED, the light source 2604 may be a green light G_LED, and the light source 2605 may be a red light R_LED. In some embodiments, the second blue light B_LED and the first blue light B_LED may have the same peak wavelength, or the peak wavelength of the second blue light B_LED may be slightly higher than the peak wavelength of the first blue light B_LED, and the UV_LED, the G_LED, and the R_LED may be the same as described above. For example, the first blue light B-LED may have a peak wavelength of 430 nm-460 nm. For example, a peak wavelength of the first blue light B-LED may be in a range of 430 nm-460 nm, a wavelength of the first blue light B-LED may be in a narrowband range, and a bandwidth of the first blue light B-LED may be about 20 nm or 30 nm. A peak wavelength of the second blue light B-LED may be in a range of 430 nm-460nm, or the peak wavelength of the second blue light B-LED may be in a range of 440 nm-470 nm, and a wavelength of the second blue light B-LED may be in a narrowband band, and a bandwidth of the second blue light B-LED may be about 20 nm or 30 nm. FIG. 28 is a schematic diagram illustrating exemplary spectrum curves of light sources according to some embodiments of the present disclosure. As shown in FIG. 28, spectrum curves of the light source 2601, the light source 2602, the light source 2603, the light source 2604, and the light source 2605 may be represented by L1, L2A, L2B, L3, and L4, respectively. The spectrum curves of L2A (e.g., the first blue light B_LED) and L2B (e.g., the second blue light B_LED) may be consistent.

FIG. 29 is a schematic diagram illustrating a transmittance spectrum of an exemplary light combination element 264 according to some embodiments of the present disclosure. As shown in FIG. 29, the light combination element 264 may have a long-wave pass characteristic with a transition zone wavelength of about 400 nm-420 nm, and reflect a spectrum of the light source 2601 (e.g., the UV_LED) that is below 410 nm and transmit a spectrum of the light source 2602 (e.g., the first blue light B_LED) that is above 410 nm, so as to realize optical path integration of a blue light emitted by the light source 2602 (e.g., the first blue light B_LED) and an ultraviolet light emitted by the light source 2601 (e.g., the UV_LED).

FIG. 7 may also be a schematic diagram illustrating a transmittance spectrum of an exemplary light combination element 263 according to some embodiments of the present disclosure. As shown in FIG. 7, the light combination element 263 may have a short-wave pass characteristic with a transition zone wavelength of about 460 nm-480 nm, which reflects a spectrum that is higher than 470 nm and transmits a spectrum of the light source 2603 (e.g., the second blue light B_LED) that is lower than 470 nm, so as to realize the optical path integration of the second blue light emitted by the light source 2603 (e.g., the second blue light B_LED) and the green light emitted by the light source 2604 (e.g., the G_LED). Meanwhile, the light combination element 263 may perform cutoff filtering on the blue excitation light in the spectrum L3 to prevent the blue excitation light from entering a subsequent optical path.

FIG. 8 may also be a schematic diagram illustrating a transmittance spectrum of an exemplary light combination element 263 according to some embodiments of the present disclosure. As shown in FIG. 8, a spectrum of the light combination element 262 may realize the optical path integration of the second blue light emitted by the light source 2603 (e.g., the second blue light B_LED), the green light emitted by the light source 2604 (e.g., the G_LED), and the red light emitted by the light source 2605 (e.g., the R_LED).

FIG. 11 may also be a schematic diagram illustrating a transmittance spectrum of an exemplary light combination element 261 according to some embodiments of the present disclosure. As shown in FIG. 11, the light combination element 261 may have a short-wave pass characteristic with a transition region wavelength of about 445 nm- 475 nm. For example, the light combination element 261 may have a short-wave pass characteristic with a transition zone wavelength of about 450 nm-460 nm, and reflect a spectrum of the second blue light emitted by the light source 2603 (e.g., the second blue light B_LED), the green light emitted by the light source 2604 (e.g., the G _LED), and the red light emitted by the light source 2605 (e.g., the R_LED) that is higher than 455 nm, and transmit a spectrum of the first blue light emitted by the light source 2602 (e.g., the first blue light B_LED) and the ultraviolet light emitted by the light source 2601 (e.g., the UV_LED) that is lower than 455 nm, thereby the optical path integration of the second blue light emitted by the light source 2603 (e.g., the second blue light B_LED), the green light emitted by the light source 2604 (e.g., the G _LED), and the red light emitted by the light source 2605 (e.g., the R_LED) with the first blue light emitted by the light source 2602 (e.g., the first blue light B_LED) and the ultraviolet light emitted by the light source 2601 (e.g., the UV_LED). As shown in FIG. 11B, the light combination element 261 may obtain the first blue light with a peak wavelength of 420 nm-455 nm by transmitting and cutting off a wavelength in the spectrum of the light source 2602 (e.g. the first blue light B_LED) that is greater than 455 nm. Meanwhile, the light combination element 261 may obtain the second blue light with a peak wavelength of 455 nm-470 nm by reflecting and cutting off a wavelength in the spectrum of the light source 2603 (e.g., the second blue light B_LED) that is less than 455 nm. The light combination element 261 may perform cutoff filtering on the long-wave spectrum of the light source 2602 (e.g., the first blue light B_LED) to obtain the first blue light with a peak wavelength of 430 nm-455 nm, and improve the contrast between the surface blood vessels and the mucosa by using a large difference in reflectivity of the spectrum below 455 nm for the surface or superficial blood vessels and the mucosa. The light combination element 261 may perform cutoff filtering on the short-wave spectrum of the light source 2603 (e.g. the second blue light B_LED) to obtain the second blue light BL2 with a peak wavelength of 455 nm-470 nm. According to a wavelength range of 450 nm-500 nm, the absorption coefficients of reduced hemoglobin and oxidized hemoglobin in the blood are greatly different, and in this range, the absorption coefficient of reduced hemoglobin is higher than the absorption coefficient of oxidized hemoglobin. Therefore, the oxygen saturation observation may be realized by reflecting the oxygen saturation in the blood based on the output image.

In some embodiments, the light source device 100 may output a hybrid light of the first blue light B-LED and the second blue light B-LED, G-LED, R-LED and UV_LED to realize ordinary light illumination, first special light illumination, and hybrid light illumination. In addition, second special light illumination with the oxygen saturation observation may also be realized.

Cutoff filtering or narrowband filtering may be performed on the light source 2601 (e.g., the UV_LED), and the components above 410 nm in the spectrum may be cut off, or narrowband filtering of ±10 nm may be performed on the spectrum of the light source 2601 (e.g., the UV_LED) with 405 nm as a center point. The light combination element 263 may perform cutoff filtering on the blue excitation light in the spectrum of the light source 2604 (e.g., the G_LED) to prevent the blue excitation light from entering the subsequent optical path, so as to facilitate the independence of each component in the output spectrum and make it easier to control the proportion of the output light, thereby achieving the stable hue of the illumination light. The light source 2602 (e.g., the first blue light B-LED) may have a first blue light wavelength with a wavelength less than 455 nm after cutoff filtering by the light combination element 261, and the light source 2603 (e.g., the second blue light B-LED) may have a second blue light wavelength with a wavelength greater than 455 nm after cutoff filtering by the light combination element 261.

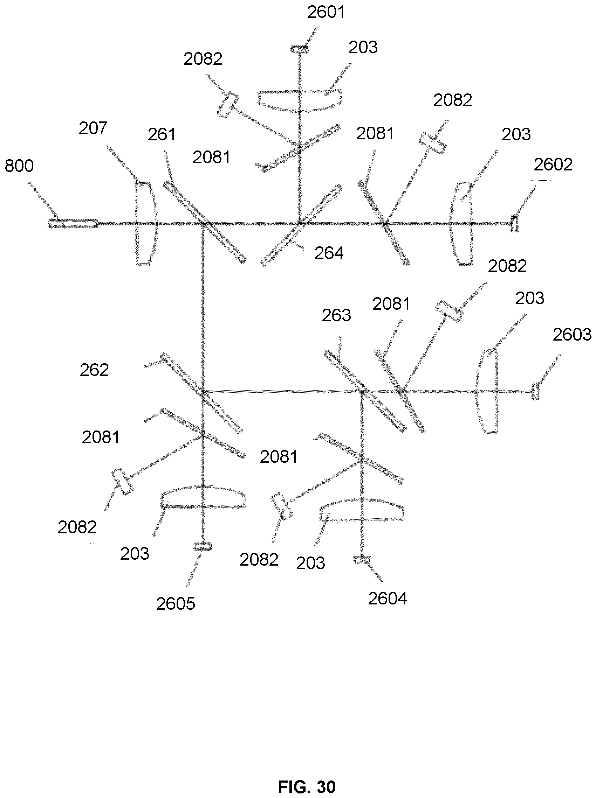

FIG. 30 is a schematic structural diagram illustrating an exemplary light source device according to some embodiments of the present disclosure. The difference between the light source device 100 shown in FIG. 30 and the light source device 100 shown in FIG. 27 is that the light combination module further includes at least one light flux measurement module 208. In some embodiments, each of the at least one light flux measurement module 208 may include the beam splitter 2081 and the photodetector 2082 corresponding to the beam splitter 2081. In some embodiments, the beam splitter 2081 may be disposed between one of light sources and one of light combination elements, and may be configured to split and reflect a beam emitted by the light source. A reflected beam may be incident into the photodetector 2082 corresponding to the beam splitter 2081. Optionally or additionally, the beam splitter 2081 may be disposed between each of the light sources (e.g., the light source 2601, the light source 2602, the light source 2603, the light source 2604, and the light source 2605) and at least one light combination element 202 (e.g., the light combination element 261, the light combination element 262, the light combination element 263, and the light combination element 264) corresponding to each of the light sources, and may be configured to split and reflect the beam emitted by the light source, and the reflected beam may be incident into the photodetector 2082 corresponding to the beam splitter 2081. The photodetector 2082 may be configured to detect a light flux of the beam incident into the photodetector 2082.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended for those skilled in the art, though not expressly stated herein. These alterations, improvements, and modifications are intended to be suggested by this disclosure and are within the spirit and scope of the exemplary embodiments of this disclosure.

Moreover, certain terminology has been used to describe embodiments of the present disclosure. For example, the terms "one embodiment," "an embodiment," and "some embodiments" mean that a particular feature, structure, or feature described in connection with the embodiment is included in at least one embodiment of the present disclosure. Therefore, it is emphasized and should be appreciated that two or more references to "an embodiment," or "one embodiment" or "an alternative embodiment" in various portions of this specification are not necessarily all referring to the same embodiment. Furthermore, the particular features, structures, or features may be combined as suitable in one or more embodiments of the present disclosure.

Furthermore, the recited order of processing elements or sequences, or the use of numbers, letters, or other designations therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose and that the appended claims are not limited to the disclosed embodiments, but, on the contrary, are intended to cover modifications and equivalent arrangements that are within the spirit and scope of the disclosed embodiments. For example, although the implementation of various parts described above may be embodied in a hardware device, it may also be implemented as a software only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. This method of disclosure, however, is not to be interpreted as reflecting an intention that the claimed subject matter requires more features than are expressly recited in each claim. Rather, claimed subject matter may lie in less than all features of a single foregoing disclosed embodiment.

**In** some embodiments, numbers describing the number of ingredients and attributes are used. It should be understood that such numbers used for the description of the embodiments use the modifier "about", "approximately", or "substantially" in some examples. Unless otherwise stated, "about," "approximately," or "substantially" indicates that the number is allowed to vary by ±20%. Correspondingly, in some embodiments, the numerical parameters used in the description and claims are approximate values, and the approximate values may be changed according to the required features of individual embodiments. In some embodiments, the numerical parameters should consider the prescribed effective digits and adopt the method of general digit retention. Although the numerical ranges and parameters used to confirm the breadth of the range in some embodiments of the present disclosure are approximate values, in specific embodiments, settings of such numerical values are as accurate as possible within a feasible range.

For each patent, patent application, patent application publication, or other materials cited in the present disclosure, such as articles, books, specifications, publications, documents, or the like, the entire contents of which are hereby incorporated into the present disclosure as a reference. The application history documents that are inconsistent or conflict with the content of the present disclosure are excluded, and the documents that restrict the broadest scope of the claims of the present disclosure (currently or later attached to the present disclosure) are also excluded. It should be noted that if there is any inconsistency or conflict between the description, definition, and/or use of terms in the auxiliary materials of the present disclosure and the content of the present disclosure, the description, definition, and/or use of terms in the present disclosure is subject to the present disclosure.

Finally, it should be understood that the embodiments described in the present disclosure are only used to illustrate the principles of the embodiments of the present disclosure. Other variations may also fall within the scope of the present disclosure. Therefore, as an example and not a limitation, alternative configurations of the embodiments of the present disclosure may be regarded as consistent with the teaching of the present disclosure. Accordingly, the embodiments of the present disclosure are not limited to the embodiments introduced and described in the present disclosure explicitly.

## Claims

1. A light source device, comprising a first light combination module and at least two light sources, wherein the light source device is configured to be connected with a light guide module, the at least two light sources include a first light source and at least one second light source, the first light combination module includes a first light combination element, wherein
the first light combination element is disposed between the light guide module and the first light source and is configured to transform a first beam into a first transmitted light, the first beam being related to the first light source;
the first light combination element is further configured to generate a first reflected light from at least one second beam emitted by the at least one second light source, combine the first reflected light and the first transmitted light into a combined light, and send the combined light to a tissue through the light guide module;
a spectrum of the first beam is narrowband or short-wavelength range.

2. The light source device of claim 1, wherein the first light combination element is configured between the first light source and the light guide module, and a distance between the first light source and an entrance of the light guide module is less than or equal to a distance between each of the at least one second light source and the entrance of the light guide module.

3. The light source device of claim 1, wherein the first light source includes any one of a violet light source, a blue light source, a green light source, an amber light source, and a red light source.

4. The light source device of claim 3, wherein the first light source is the violet light source configured to emit a short-wave spectrum, a peak wavelength of the at least one second light source is longer than a peak wavelength of the violet light source, and the first light combination element is a short-wave pass dichroic mirror.

5. The light source device of claim 3, wherein the first light source is the red light source configured to emit a narrowband spectrum, a peak wavelength of the at least one second light source is shorter than a peak wavelength of the red light source, and the first light combination element is a long-wave pass dichroic mirror.

6. The light source device of any one of claims 1-5, wherein when the number of the at least one second light source N is at least two, the first light combination module also includes at least N-1 second light combination elements, and each of the N-1 second light combination elements is configured to combine a second beam emitted by each of the at least two second light sources by reflecting and/or transmitting the second beam emitted by each of the at least two second light sources to generate a first incident beam, and the first incident beam is incident onto the first light combination element for reflection.

7. The light source device of claim 6, wherein the first light combination element and/or at least one of the second light combination elements combine the first beam and/or the second beam emitted by a corresponding first light source and/or corresponding second light source and perform long-wave cutoff filtering, short-wave cutoff filtering, or narrowband filtering on the first beam and/or the second beam.

8. The light source device of claim 6, wherein the first light source is a broadband amber light source, the first light combination element is a dichroic mirror, and the first beam emitted by the first light source is filtered by the dichroic mirror to obtain a narrowband light with a wavelength being in a range of 590 nm-610 nm or a range of 595 nm-610 nm.

9. The light source device of claim 6, further comprising a filter switch-in and switch-out module, wherein the filter switch-in and switch-out module includes a filter configured to switch between a filter switch-in mode and a filter switch-out mode, and bandwidths of a light output at a position of the filter in the filter cut-in mode and the filter switch-out mode are different.

10. The light source device of claim 9, wherein the at least one second light source includes a green light source with a peak wavelength within a range of 510 nm-560 nm, the filter switch-in and switch-out module includes a green light filter, and the green light filter is configured to filter a light emitted by the green light source to obtain a narrowband green light in a range of 520 nm-550 nm or a range of 530 nm-550 nm.

11. The light source device of claim 6, wherein the first light combination element or each of the second light combination elements includes a dichroic mirror, the dichroic mirror is provided with a first optical surface and a second optical surface, after each light source emits light, the first optical surface is configured to perform optical path integration on corresponding light to output a combined light, and the second optical surface is configured to detect a light flux after splitting the corresponding light.

12. The light source device of claim 11, further comprising at least one light flux measurement module configured to detect the light, wherein a position of the at least one light flux measurement module corresponds to a position of a light source to be detected;
the second optical surface is capable of splitting light emitted by a corresponding light source to obtain a reflected light to enter the at least one light flux measurement module as a detection light.

13. The light source device of claim 12, wherein the first optical surface of the dichroic mirror is capable of outputting the combined light after the optical path integration, or
the first optical surface of the dichroic mirror is capable of transmitting light incident onto the first optical surface to perform light flux detection.

14. The light source device of any one of claims 11-13, wherein a first optical zone and a second optical zone are disposed on the first optical surface of the dichroic mirror, a ratio of an area of the first optical zone to an area of the first optical surface is greater than or equal to 90%, and a ratio of an area of the second optical zone to the area of the first optical surface is less than or equal to 10%;
the second optical zone is configured to transmit light emitted by a corresponding light source such that light enters a corresponding light flux measurement module of the at least one light flux measurement module.

15. The light source device of claim 14, wherein a dichroic filter film is disposed on the first optical zone,
the second optical zone satisfies at least one condition of:
a beam splitting film being disposed on the second optical zone,
an antireflection film being disposed on the second optical zone, or
the second optical zone being uncoated.

16. The light source device of claim 14, wherein a photosensitive surface of a light flux measurement module of the at least one light flux measurement module corresponding to the light transmitted through the second optical zone faces a direction of a detection optical axis of the dichroic mirror transmitted through the second optical zone.

17. The light source device of claim 14, wherein a size of a light beam transmitted to the corresponding light flux measurement module of the at least one light flux measurement module through the second optical zone is greater than a size of a photosensitive surface.

18. The light source device of claim 12, wherein a photosensitive surface of a light flux measurement module of the at least one light flux measurement module corresponding to light reflected by the second optical surface is perpendicular to a direction of a detection optical axis of the dichroic mirror reflected by the second optical surface.

19. The light source device of claim 18, wherein a size of a beam reflected to the photosensitive surface of the light flux measurement module of the at least one light flux measurement module by the second optical surface is less than a size of a beam of a detection light on the corresponding light source.

20. The light source device of claim 11, wherein a beam splitting film is disposed on the first optical surface of the dichroic mirror, and the beam splitting film is capable of transmitting light emitted by a corresponding light source while performing optical path integration through reflection, or the beam splitting film is capable of reflecting the light emitted by the corresponding light source while performing optical path integration through transmission, such that a transmitted or reflected light enters a corresponding light flux measurement module.

21. The light source device of claim 11, wherein a dichroic filter film is disposed on the first optical surface of the dichroic mirror.

22. The light source device of claim 12, further comprising a background light detector, wherein the background light detector is located outside a range covered by a detection beam on a corresponding light flux measurement module of the at least one light flux measurement module.

23. The light source device of claim 12, wherein the at least one light flux measurement module includes an opening aperture disposed at a front end.

24. The light source device of claim 11, comprising a plurality of dichroic mirrors, wherein a beam splitting film is disposed on a second optical surface of each of the plurality of dichroic mirrors, the beam splitting film is configured to split light emitted by a corresponding light source, and each beam splitting film is configured to split a light of a different wavelength, a splitting wavelength range of the beam splitting film being determined by a wavelength of the light.

25. The light source device of claim 11, comprising a plurality of dichroic mirrors, wherein a same beam splitting film is disposed on a second optical surface of each of the plurality of dichroic mirrors, the same beam splitting film is configured to split a light reflected by the second optical surface, and when the light reflected by the second optical surface has different wavelengths, a beam splitting wavelength range of the same beam splitting film is capable of covering the wavelength of the light with the different wavelengths.

26. The light source device of claim 24 or claim 25, wherein the beam splitting film is capable of reflecting less than or equal to 10% of light and transmitting greater than or equal to 90% of light.

27. The light source device of claim 6, wherein the first light source is the violet light source configured to emit a short-wave spectrum, a peak wavelength of the at least one second light source is longer than a peak wavelength of the violet light source, a peak wavelength of a second beam emitted by the at least one second light source and reflected by the at least one second light combination element is longer than a peak wavelength of the second beam emitted by the at least one second light source and transmitted by the at least one second light combination element, the second light combination element is a short-wave pass dichroic mirror.

28. The light source device of claim 6, wherein the first light source is the red light source configured to emit a narrowband spectrum, a peak wavelength of the at least one second light source is less than a peak wavelength of the red light source, a peak wavelength of a second beam emitted by the at least one second light source and reflected by the at least one second light combination element is less than a peak wavelength of a second beam emitted by the at least one second light source and transmitted by the at least one second light combination element, the second light combination element being a long-wave pass dichroic mirror.

29. The light source device of any one of claims 6-28, wherein a first angle between each of the at least one second light combination element and the first light combination element is less than a first preset angle.

30. The light source device of any one of claims 6-29, wherein a second angle between the first light combination element and an optical axis where the first light combination element is located is greater than or equal to a second preset angle and less than or equal to a third preset angle; and a third angle between each of the at least one second light combination element and an optical axis where the second light combination element is located is greater than or equal to a fourth preset angle and less than or equal to a fifth preset angle.

31. The light source device of claim 30, wherein the second preset angle or the fourth preset angle is 40°, and the third preset angle or the fifth preset angle is 50°.

32. The light source device of any one of claims 6-31, further comprising a light guide component, wherein
the light guide component is disposed between the first light source and the first light combination element and configured to transmit the first beam to the first light combination element;
the light guide component is disposed between one of the at least one second light source and a corresponding second light combination element and configured to transmit the second beam to the corresponding second light combination element; or
the light guide component is disposed between the first light combination element and the light guide module and configured to transmit the combined light to the light guide module.

33. The light source device of any one of claims 6-32, wherein the first light combination module further includes a collimating lens;
the collimating lens is disposed between the first light source and the first light combination element and configured to convert the first beam into a parallel beam to be incident onto the first light combination element; and/or
the collimating lens is disposed between one of the at least one second light source and a corresponding second light combination element and configured to convert the second beam into a parallel beam to be incident onto the corresponding second light combination element.

34. The light source device of any one of claims 6-33, wherein the first light combination module further includes a first filter and/or a second filter;
the first filter is disposed between the first light source and the first light combination element and configured to transmit a beam in a first target waveband of the light emitted by the first light source;
the second filter is disposed between one of the at least one second light source and a corresponding second light combination element and configured to transmit a beam in a second target waveband of the second beam.

35. The light source device of claim 34, wherein the second filter is disposed between one of the at least one second light source and the corresponding second light combination element, and an optical axis of the second light source is parallel to an optical axis of an output light from the light guide module.

36. The light source device of any one of claims 1-35, wherein the first light combination module further includes a focusing lens, the focusing lens is disposed between the first light combination element and the light guide module and configured to focus the combined light to obtain a focused beam coupled into the light guide module.

37. The light source device of any one of claims 1-36, further comprising at least one light flux measurement module, wherein the at least one light flux measurement module includes a beam splitter and a photodetector corresponding to the beam splitter;
the beam splitter is disposed between the first light source and the first light combination element and configured to split the first beam to obtain a third beam, and reflect the third beam to the photodetector corresponding to the beam splitter; and/or,
the beam splitter is disposed between each of the at least one second light source and a second light combination element corresponding to the second light source and configured to split the second beam to obtain a fourth beam, and reflect the fourth beam to the photodetector corresponding to the beam splitter;
the photodetector is configured to detect a light flux of the third beam and/or the fourth beam incident into the photodetector.

38. The light source device of claim 37, wherein an angle between a reflection surface of the beam splitter and an optical axis where the beam splitter is located is in a range of 50°-70°; or an angle between the beam splitter and the first light combination element or the second light combination element corresponding to the beam splitter is less than a sixth preset angle.

39. The light source device of claim 37, wherein the at least one light flux measurement module further includes a third filter;
the third filter is disposed between the beam splitter and the photodetector corresponding to the beam splitter and configured to transmit a beam in a third target waveband and/or a fourth target waveband;
a difference between the third target waveband and the first target waveband of the first beam in the combined light is less than a first preset difference threshold; and
a difference between the fourth target waveband and the second target waveband of the second beam in the combined light is less than a second preset difference threshold.

40. The light source device of any one of claims 1-39, further comprising a first heat dissipation module and a second heat dissipation module for heat dissipation from the light source device; wherein a heat dissipation direction of the first heat dissipation module is parallel to an optical axis of an output light, and a heat dissipation direction of the second heat dissipation module is perpendicular to the optical axis of the output light.

41. The light source device of any one of claims 1-40, further comprising a light source extension interface, wherein the light source extension interface is configured to connect an extension module; the extension module includes at least one third light source and a second light combination module corresponding to the at least one third light source, the second light combination module corresponding to the at least one third light source includes a third light combination element, the third light combination element is configured to reflect and/or transmit a fifth beam emitted by the at least one third light source to realize light combination in sequence, so as to generate a second incident beam that is incident onto a second light combination element for reflection or transmission.

42. The light source device of claim 41, wherein the at least one third light source of the extension module includes a red light source.

43. The light source device of claim 41, wherein the at least one third light source of the extension module includes a red light source and an amber light source.

44. The light source device of claim 41, wherein the at least one third light source includes a red light source, a first infrared light source, and a second infrared light source.

45. The light source device of claim 41, wherein a beam associated with the at least one second light source or the at least one third light source has a narrowband spectrum or a short-wave spectrum, and an optical axis of the at least one second light source or the at least one third light source is parallel to an optical axis of an output light of the light guide module.

46. An endoscope system, comprising a light guide module, a lighting module, a camera module, a processing module, a display module, and a light source device of any one of claims 1-45; wherein
the light source device is configured to input the combined light into the light guide module;
the light guide module is configured to transmit an input light to the lighting module;
the lighting module is configured to diffuse the combined light transmitted to the lighting module to a tissue;
the camera module is configured to obtain an image of the tissue;
the processing module is configured to perform signal processing on the image to obtain an image; and
the display module is configured to display the image after the signal processing.

47. The endoscope system of claim 46, wherein the light source device includes a photodetector;
the photodetector is configured to detect a light flux of each light source;
the processing module is further configured to obtain a detection signal of the light flux, and adjust a driving current of the light source device based on a difference value between the detection signal and a preset detection signal.

48. The endoscope system of claim 46 or claim 47, further comprising an input module and a control module; wherein
the input module is configured to obtain an input instruction, the input instruction including an operation instruction for any one of an ordinary white light mode, a special light mode, and a hybrid light mode;
the control module is configured to control a light mode of the combined light output from the light source device based on any one of the ordinary white light mode, the special light mode, and the hybrid light mode in the input instruction.
